# EUROPEAN PATENT SPECIFICATION

(11) **EP 0 476 130 B1**
(45) Date of publication and mention of the grant of the patent: **09.07.2008**
(21) Application number: 91908163.8
(22) Date of filing: 05.04.1991
(51) Int. Cl.: C12N 15/51, C07K 14/08, G01N 33/576, C12Q 1/70, A61K 39/29, C12N 7/02

(54) **ENTERICALLY TRANSMITTED NON-A/NON-B HEPATITIS VIRAL AGENT AND CHARACTERISTIC EPITOPES THEREOF**
ENTERISCH ÜBERTRAGENDES NON-A/NON-B-HEPATITIS-VIRUS AGENS UND CHARAKTERISTISCHE EPITOPE VON SELBIGEM
AGENT VIRAL D'HEPATITE NON A/NON B TRANSMIS PAR VOIE ENTERIQUE ET EPITOPES CARACTERISTIQUES DE CET AGENT

(30) Priority: 05.04.1990 US 505888
(43) Date of publication of application: 25.03.1992
(73) Proprietor: GENELABS TECHNOLOGIES, INC., Redwood City, CA 94063 (US); GOVERNMENT OF THE UNITED STATES OF AMERICA, as repr. by THE SECR. OF THE DEPT. OF HEALTH AND HUMAN SERVICES AND HIS SUCCESSORS, Rockville, MD 20852 (US)
(72) Inventor: REYES, Gregory, R., Palo Alto, CA 94303 (US); YARBOUGH, Patrice, O., Redwood Shores, CA 94065 (US); BRADLEY, Daniel, W., Lawrenceville, GA 30244 (US); KRAWCZYNSKI, Krzysztof, Z., Tucker, GA 30084 (US); TAM, Albert, San Francisco, CA 94122 (US); FRY, Kirk, E., Palo Alto, CA 94306 (US)
(74) Representative: Harrison, David Christopher
(86) International application number: PCT/US1991/002368
(87) International publication number: WO 1991/015603

(56) References cited:
- WO-A1-85/01517
- WO-A1-89/12462
- WO-A1-89/12641
- US-A- 4 659 678
- US-A- 4 683 195
- US-A- 4 871 659
- SCIENCE vol. 247, 16 March 1990, AAAS,WASHINGTON,DC,US; pages 1335 - 1339 G.R. REYES ET AL. 'Isolation of a cDNA from the virus responsible for enterically transmitted non-A, non-B hepatitis'
- INTERNATIONAL CONGRESS SERIES vol. 895, 1991, ELSEVIER SCIENCE, AMSTERDAM, NL; T. SHIKATA ET AL. (ED.); 'Viral hepatitis C, D and E' CHAPTER 43 : Hepatitis E virus HEV: epitope mapping and detection of strain variation; G.R. REYES ET AL.; pages 237-245 Proceedings of the international meeting on Non-A, non-B hepatitis, Tokyo, 27-30 September 1989, Japan;

## Description

### CROSS-REFERENCE TO RELATED APPLICATIONS

This application is a continuation-in-part of U.S. Application Serial No.07/505,888, filed April 5, 1990, which is a continuation-in-part of U.S. Application Serial No. 420,921, filed October 13, 1989, which is a continuation-in-part of U.S. Application Serial No. 367,486, filed June 16, 1989, which is a continuation-in-part of U.S. Application Serial No. 336,672, filed April 11, 1989, which is a continuation-in-part of U.S. Application Serial No. 208,997, filed June 17, 1988, all of which are herein incorporated by reference.

### INTRODUCTION

### Field of Invention

This invention relates to recombinant proteins, genes, and gene probes and more specifically to such proteins and probes derived from an enterically transmitted nonA/nonB hepatitis viral agent, to diagnostic methods and vaccine applications which employ the proteins and probes, and to gene segments that encode specific epitopes (and proteins artificially produced to contain those epitopes) that are particularly useful in diagnosis and prophylaxis.

### Background

Enterically transmitted non-A/non-B hepatitis viral agent (ET-NANB; also referred to herein as HEV) is the reported cause of hepatitis in several epidemics and sporadic cases in Asia, Africa, Europe, Mexico, and the Indian subcontinent. Infection is usually by water contaminated with feces, although the virus may also spread by close physical contact. The virus does not seem to cause chronic infection. The viral etiology in ET-NANB has been demonstrated by infection of volunteers with pooled fecal isolates; immune electron microscopy (IEM) studies have shown virus particles with 27-34 nm diameters in stools from infected individuals. The virus particles reacted with antibodies in serum from infected individuals from geographically distinct regions, suggesting that a single viral agent or class is responsible for the majority of ET-NANB hepatitis seen worldwide. No antibody reaction was seen in serum from individuals infected with parenterally transmitted NANB virus (also known as hepatitis C virus or HCV), indicating a different specificity between the two NANB types.

In addition to serological differences, the two types of NANB infection show distinct clinical differences. ET-NANB is characteristically an acute infection, often associated with fever and arthralgia, and with portal inflammation and associated bile stasis in liver biopsy specimens (Arankalle). Symptoms are usually resolved within six weeks. Parenterally transmitted NANB, by contrast, produces a chronic infection in about 50% of the cases. Fever and arthralgia are rarely seen, and inflammation has a predominantly parenchymal distribution (Khuroo, 1980). The course of ET-NANBH is generally uneventful in healthy individuals, and the vast majority of those infected recover without the chronic sequelae seen with HCV. One peculiar epidemiologic feature of this disease, however, is the markedly high mortality observed in pregnant women; this is reported in numerous studies to be on the order of 10-20%. This finding has been seen in a number of epidemiologic studies but at present remains unexplained. Whether this reflects viral pathogenicity, the lethal consequence of the interaction of virus and immune suppressed (pregnant) host, or a reflection of the debilitated prenatal health of a susceptible malnourished population remains to be clarified.

The two viral agents can also be distinguished on the basis of primate host susceptibility. ET-NANB, but not the parenterally transmitted agent, can be transmitted to cynomolgus monkeys. The parenterally transmitted agent is more readily transmitted to chimpanzees than is ET-NANB (Bradley, 1987).

There have been major efforts worldwide to identify and clone viral genomic sequences associated with ET-NANB hepatitis. One goal of this effort, requiring virus-specific genomic sequences, is to identify and characterize the nature of the virus and its protein products. Another goal is to produce recombinant viral proteins which can be used in antibody-based diagnostic procedures and for a vaccine. Despite these efforts, viral sequences associated with ET-NANB hepatitis have generally not been successfully identified or cloned heretofore, nor have any virus-specific proteins been identified or produced. PCT Publication WO 89/12462 and Reyes et al . (Science, 1990, 247: 1335-1339) disclose proteins derived from an enterically transmitted non-A/non-B viral hepatitis agent whose genome contains a region homologous to a coding region of the 1.33 kb DNA EcoRI insert present in plasmid pTZKF1 (ET1.1) carried in *E.coli* strain BB4 and having ATCC deposit No. 67717.

### Relevant Literature

Arankalle, V.A., et al., The Lancet, 550 (March 12, 1988).

Bradley, D.W., et al., J Gen. Virol., 69:1 (1988).

Bradley, D.W. et al., Proc. Nat. Acad. Sci., USA, 84:6277 (1987).

Gravelle, C.R. et al., J. Infect. Diseases, 131:167 (1975).

Kane, M.A., et al., JAMA, 252:3140 (1984).

Khuroo, M.S., Am. J. Med., 48:818 (1980).

Khuroo, M.S., et al., Am. J. Med., 68:818 (1983).

Maniatis, T., et al. Molecular Cloning: A Laboratory Manual, Cold Spring Harbor Laboratory (1982).

Seto, B., et al., Lancet, 11:941 (1984).

Sreenivasan, M.A., et al., J. Gen. Virol., 65:1005 (1984).

Tabor, E., et al., J. Infect. Dis., 140:789 (1979).

### SUMMARY OF THE INVENTION

The invention is as defined in the accompanying claims. Novel compositions, as well as methods of preparation and use of the compositions are provided, where the compositions comprise viral proteins and fragments thereof derived from the viral agent for ET-NANB. A number of specific fragments of viral proteins (and the corresponding genetic sequences) that are particularly useful in diagnosis and vaccine production are also disclosed. Methods for preparation of ET-NANB viral proteins include isolating ET-NANB genomic sequences which are then cloned and expressed in a host cell. The resultant recombinant viral proteins find use as diagnostic agents and as vaccines. The genomic sequences and fragments thereof find use in preparing ET-NANB viral proteins and as probes for virus detection.

### BRIEF DESCRIPTION OF THE DRAWINGS

Figure 1 shows vector constructions and manipulations used in obtaining and sequencing cloned ET-NANB fragment; and
Figures 2A-2B are representations of Southern blots in which a radiolabeled ET-NANB probe was hybridized with amplified cDNA fragments prepared from RNA isolated from infected (I) and non-infected (N) bile sources (2A), and from infected (I) and non-infected (N) stool-sample sources (2B).

### DESCRIPTION OF SPECIFIC EMBODIMENTS

Novel compositions comprising generic sequences and fragments thereof derived from the viral agent for ET-NANB are provided, together with recombinant viral proteins produced using the genomic sequences and methods of using these compositions. Epitopes on the viral protein have been identified that are particularly useful in diagnosis and vaccine production. Small peptides containing the epitopes are recognized by multiple sera of patients infected with ET-NANB.

The molecular cloning of HEV was accomplished by two very different approaches. The first successful identification of a molecular clone was based on the differential hybridization of putative HEV cDNA clones to heterogeneous cDNA from infected and uninfected cyno bile. cDNAs from both sources were labeled to high specific activity with ³²P to identify a clone that hybridized specifically to the infected source probe. A cyno monkey infected with the Burma isolate of HEV was used in these first experiments. The sensitivity of this procedure is directly related to the relative abundance of the specific sequence against the overall background. In control experiments, it was found that specific identification of a target sequence may be obtained with as little as 1 specific part per 1000 background sequences. A number of clones were identified by this procedure using libraries and probes made from infected (Burma isolate) and control uninfected cyno bile. The first extensively characterized clone of the 16 plaques purified by this protocol was given the designation ET1.1.

ET1.1 was first characterized as both derived from and unique to the infected source cDNA. Heterogeneous cDNA was amplified from both infected and uninfected sources using a sequence independent single premier amplification technique (SISPA). This technique is described in copending application serial No. 208,512, filed June 17, 1988. The limited pool of cDNA made from Burma infected cyno bile could then be amplified enzymatically prior to cloning or hybridization using putative HEV clones as probes. ET1.1 hybridized specifically to the original bile cDNA from the infected source. Further validation of this clone as derived from the genome of HEV was demonstrated by the similarity of the ET1.1 sequence and those present in SISPA cDNA prepared from five different human stool samples collected from different ET-NANBH epidemics including Somalia, Tashkent, Borneo, Mexico and Pakistan. These molecular epidemiologic studies established the isolated sequence as derived from the virus that represented the major cause of ET-NANBH worldwide.

The viral specificity of ET1.1 was further established by the finding that the clone hybridized specifically to RNA extracted from infected cyno liver. Hybridization analysis of polyadenylated RNA demonstrated a unique 7.5 Kb polyadenylated transcript not present in uninfected liver. The size of this transcript suggested that it represented the full length viral genome. Strand specific oligonucleotides were also used to probe viral genomic RNA extracted directly from semi-purified virions prepared from human stool. The strand specificity was based on the RNA-directed RNA polymerase (RDRP) open reading frame (ORF) identified in ET1.1 (see below). Only the probe detecting the sense strand hybridized to the nucleic acid. These studies characterized HEV as a plus sense, single stranded genome. Strand specific hybridization to RNA extracted from the liver also established that the vast majority of intracellular transcript was positive sense. Barring any novel mechanism for virus expression, the negative strand, although not detectable, would be present at a ratio of less than 1:100 when compared with the sense strand.

ET1.1 was documented as exogenous when tested by both Southern blot hybridization and PCR using genomic DNAs derived from uninfected humans, infected and uninfected cynos and also the genomic DNAs from E. coli and various bacteriophage sources. The latter were tested in order to rule out trivial contamination with an exogenous sequence introduced during the numerous enzymatic manipulations performed during cDNA construction and amplification. It was also found that the nucleotide sequence of the ET1.1 clone was not homologous to any entries in the Genebank database. The translated open reading frame of the ET1.1 clone did, however, demonstrate limited homology with consensus amino acid residues consistent with an RNA-directed RNA polymerase. This consensus amino acid motif is shared among all positive strand RNA viruses and, as noted above, is present at the 3' end of the HCV genome. The 1.3 Kb clone was therefore presumed to be derived, at least in part, from the nonstructural portion of the viral genome.

Because of the relationship of different strains of ET-NANB to each other that has been demonstrated by the present invention, the genome of the ET-NANB viral agent is defined in this specification as containing a region which is homologous to the 1.33 kb DNA EcoRI insert present in plasmid pTZKF1 (ET1.1) carried in E. coli strain BB4 and having ATCC deposit no. 67717. The entire sequence, in both directions, has now been identified as set forth below. The sequences of both strands are provided, since both strands can encode proteins. However, the sequence in one direction has been designated as the "forward" sequence because of statistical similarities to known proteins and because the forward sequence is known to be predominately protein-encoding. This sequence is set forth below along with the three possible translation sequences. There is one long open reading frame that starts at nucleotide 145 with an isoleucine and extends to the end of the sequence. The two other reading frames have many termination codons. Standard abbreviations for nucleotides and amino acids are used here and elsewhere in this specification.

The gene sequence given below is substantially identical to one given in the parent application. The present sequence differs in the omission of the first 37 nucleotides at the 5' end and last 13 nucleotides at the 3' end, which are derived from the linker used for cloning rather than from the virus. In addition, a G was omitted at position 227 of the sequence given in the parent application.

The following gene sequence has SEQ ID NO.1; the first amino acid sequence in reading frame beginning with nucleotide 1 has SEQ ID NO.2; the second amino acid sequence in reading frame beginning with nucleotide 2 has SEQ ID NO.3; and the third amino acid sequence in reading frame beginning with nucleotide 3 has SEQ ID NO.4.

### Forward Sequence

The complementary strand, referred to here as the "reverse sequence," is set forth below in the same manner as the forward sequence set forth above. Several open reading frames, shorter than the long open reading frame found in the forward sequence, can be seen in this reverse sequence. Because of the relative brevity of the open reading frames in the reverse direction, they are probably not expressed.

The following gene sequence has SEQ ID NO.5.

### Reverse Sequence

Identity of this sequence with sequences in etiologic agents has been confirmed by locating a corresponding sequence in a viral strain isolated in Burma. The Burmese isolate contains the following sequence of nucleotides (one strand and open reading frames shown). The following gene sequence has SEQ ID NO.6; the protein sequence corresponding to ORF1 has SEQ ID NO.7; ORF2 has SEQ ID NO.8; and ORF3 has SEQ ID NO.9.

Total number of bases in this sequence as presented is 7195. The poly-A tail present in the cloned sequence has been omitted.

The ability of the methods described herein to isolate and identify genetic material from other NANB hepatitis strains has been confirmed by identifying genetic material from an isolate obtained in Mexico. The sequence of this isolate was about 75% identical to the ET1.1 sequence set forth in SEQ ID NO.1 above. The sequence was identified by hybridization using the conditions set forth in Section II.B below.

In this different approach to isolation of the virus, cDNA libraries were made directly from a semi-purified human stool specimen collected from an outbreak of ET-NANB in Telixtac. The recovery of cDNA and the construction of representative libraries was assured by the application of sequence independent single premier amplification (SISPA). A cDNA library constructed in lambda gt11 from such an amplified cDNA population was screened with a serum considered to have "high" titer anti-HEV antibodies as assayed by direct immunofluorescence on liver sections from infected cynos. Two cDNA clones, denoted 406.3-2 and 406.4-2, were identified by this approach from a total of 60,000 screened. The sequence of these clones was subsequently localized to the 3' half of the viral genome by homology comparison to the HEV (Burma) sequence obtained from clones isolated by hybridization screening of libraries with the original ET1.1 clone.

These isolated cDNA epitopes when used as hybridization probes on Northern blots of RNA extracted from infected cyno liver gave a somewhat different result when compared to the Northern blots obtained with the ET1.1 probe. In addition to the single 7.5 Kb transcript seen using ETI.1, two additional transcripts of 3.7 and 2.0 Kb were identified using either of these epitopes as hybridization probes. These polyadenylated transcripts were identified using the extreme 3' end epitope clone (406.3-2) as probe and therefore established these transcripts as co-terminal with the 3' end of the genome (see below). One of the epitope clones (406.4-2) was subsequently shown to react in a specific fashion with antisera collected from 5 different geographic epidemics (Somalia, Burma, Mexico, Tashkent and Pakistan). The 406.3-2 clone reacted with sera from 4 out of these same 5 epidemics (Yarbough et al., 1990). Both clones reacted with only post inoculation antisera from infected cynos. The latter experiment confirmed that seroconversion in experimentally infected cynos was related to the isolated exogenous cloned sequence.

A composite cDNA sequence (obtained from several clones of the Mexican strain) is set forth below. Composite Mexico strain sequence (SEQ ID NO.10):

The above sequence was obtained from polyadenylated clones. For clarity the 3' polyA "tail" has been omitted.

The sequence above includes a partial cDNA sequence consisting of 1661 nucleotides that was identified in a previous application in this series. The previously identified partial sequence is set forth below, with certain corrections (SEQ ID NO.11). The corrections include deletion of the first 80 bases of the prior reported sequence, which are cloning artifacts; insertion of G after former position 174, of C after 270, and of GGCG after 279; change of C to T at former position 709, of GC to CG at 722-723, of CC to TT at 1238-39, and of C to G at 1606; deletion of T at former position 765; and deletion of the last 11 bases of the former sequence, which are part of a linker sequence and are not of viral origin.

Non-A Non-B T: Mexican Strain; SEQ ID NO.11

When comparing the Burmese and Mexican strains, 75.7% identity is seen in a 7189 nucleotide overlap beginning at nucleotide 1 of the Mexican strain and nucleotide 25 of the Burmese strain.

In the same manner, a different strain of HEV was identified in an isolate obtained in Tashkent, U.S.S.R. The Tashkent sequence is given below (SEQ ID NO.12):

As shown in the following comparison of sequences, the Tashkent (Tash.) sequence more closely resembles the Burma sequence than the Mexico sequence, as would be expected of two strains from more closely related geographical areas. The numbering system used in the comparison is based on the Burma sequence. As indicated previously, Burma has SEQ ID NO:6; Mexico, SEQ ID NO:10; and Tashkent, SEQ ID NO:12. The letters present in the lines between the sequences indicate conserved nucleotides.

A number of open reading frames, which are potential coding regions, have been found within the DNA sequences set forth above. As has already been noted, consensus residues for the RNA-directed RNA polymerase (RDRP) were identified in the HEV (Burma) strain clone ET1.1. Once a contiguous overlapping set of clones was accumulated, it became clear that the nonstructural elements containing the RDRP as well as what were identified as consensus residues for the helicase domain were located in the first large open reading frame (ORFI). ORFI covers the 5' half of the genome and begins at the first encoded met, after the 27th bp of the apparent non-coding sequence, and then extends 5079 bp before reaching a termination codon. Beginning 37 bp downstream from the ORF1 stop codon in the plus 1 frame is the second major opening reading frame (ORF2) extending 1980 bp and terminating 68 bp upstream from the point of poly A addition. The third forward ORF (in the plus 2 frame) is also utilized by HEV. ORF3 is only 370 bp in length and would not have been predicted to be utilized by the virus were it not for the identification of the immunoreactive cDNA clone 406.4-2 from the Mexico SISPA cDNA library (see below for detailed discussion). This epitope confirmed the utilization of ORF3 by the virus, although the means by which this ORF is expressed has not yet been fully elucidated. If we assume that the first met is utilized, ORF3 overlaps ORF1 by 1 bp at its 5' end and ORF2 by 328 bp at its 3'end. ORF2 contains the broadly reactive 406.3-2 epitope and also a signal sequence at its extreme 5' end. The first half of this ORF2 also has a high pI value (>10) similar to that seen with other virus capsid proteins. These data suggest that the ORF2 might be the predominant structural gene of HEV.

The existence of subgenomic transcripts prompted a set of experiments to determine whether these RNAs were produced by splicing from the 5' end of the genome. An analysis using subgenomic probes from throughout the genome, including the extreme 5' end, did not provide evidence for a spliced transcript. However, it was discovered that a region of the genome displayed a high degree of homology with a 21 bp segment identified in Sindbis as a probably internal initiation site for RNA transcription used in the production of its subgenomic messages. Sixteen of 21 (76%) of the nucleotides are identical.

Two cDNA clones which encode an epitope of HEV that is recognized by sera collected from different ET-NANB outbreaks (i.e., a universally recognized epitope) have been isolated and characterized. One of the clones immunoreacted with 8 human sera from different infected individuals and the other clone immunoreacted with 7 of the human sera tested. Both clones immunoreacted specifically with cyno sera from infected animals and exhibited no immunologic response to sera from uninfected animals. The sequences of the cDNAs in these recombinant phages, designated 406.3-2 and 406.4-2 have been determined. The HEV open reading frames are shown to encode epitopes specifically recognized by sera from patients with HEV infections. The cDNA sequences and the polypeptides that they encode are set forth below. Epitopes derived from Mexican strain of HEV:

406.4-2 sequence (nucleotide sequence has SEQ ID NO.13; amino acid sequence has SEQ ID NO.14):

The universal nature of these epitopes is evident from the homology exhibited by the DNA that encodes them. If the epitope coding sequences from the Mexican strains shown above are compared to DNA sequences from other strains, such as the Burmese strain also set forth above, similarities are evident, as shown in the following comparisons.

### Comparison of 406.4-2 epitopes, HEV Mexico and Burma strains:

There is 73.5% identity in a 33-amino acid overlap.

### Comparison of 406.3-2 epitopes, HEV Mexico and Burma strains: MEXICAN(SEQ ID No.19)

BURMA(SEQ ID No.20)
There is 90.5% identity in the 42-amino acid overlap.

It will be recognized by one skilled in the art of molecular genetics that each of the specific DNA sequences given above shows a corresponding complementary DNA sequence as well as RNA sequences corresponding to both the principal sequence shown and the complementary DNA sequence. Additionally, open reading frames encoding peptides are present, and expressible peptides are disclosed by the nucleotide sequences without setting forth the amino acid sequences explicitly, in the same manner as if the amino acid sequences were explicitly set forth as in the ET1.1 sequence or other sequences above.

### DETAILED DESCRIPTION OF THE INVENTION

### I. Definitions

The terms defined below have the following meaning herein:
1. "Enterically transmitted non-A/non-B hepatitis viral agent, ET-NANB, or HEV" means a virus, virus type, or virus class which (1) causes waterborne, infectious hepatitis, (ii) is transmissible in cynomolgus monkeys, (iii) is serologically distinct from hepatitis A virus (HAV), hepatitis B virus (HBV), hepatitis C virus (HCV), and hepatitis D virus, and (iv) includes a genomic region which is homologous to the 1.33 kb cDNA insert in plasmid pTZKF1(ET1.1) carried in E. coli strain BB4 identified by ATCC deposit number 67717.
2. Two nucleic acid fragments are "homologous" if they are capable of hybridizing to one another under hybridization conditions described in Maniatis et al., op. cit., pp. 320-323. However, using the following wash conditions: 2 x SCC, 0.1% SDS, room temperature twice, 30 minutes each; then 2 x SCC, 0.1% SDS, 50°C once, 30 minutes; then 2 x SCC, room temperature twice, 10 minutes each, homologous sequences can be identified that contain at most about 25-30% basepair mismatches. More preferably, homologous nucleic acid strands contain 15-25% basepair mismatches, even more preferably 5-15% basepair mismatches. These degrees of homology can be selected by using more stringent wash conditions for identification of clones from gene libraries (or other sources of genetic material), as is well known in the art.
3. Two amino acid sequences or two nucleotide sequences (in an alternative definition for homology between two nucleotide sequences) are considered homologous (as this term is preferably used in this specification) if they have an alignment score of >5 (in standard deviation units) using the program ALIGN with the mutation gap matrix and a gap penalty of 6 or greater. See Dayhoff, M.O., in Atlas of Protein Sequence and Structure (1972) Vol. 5, National Biomedical Research Foundation, pp. 101-110, and Supplement 2 to this volume, pp. 1-10. The two sequences (or parts thereof, preferably at least 30 amino acids in length) are more preferably homologous if their amino acids are greater than or equal to 50% identical when optimally aligned using the ALIGN program mentioned above.
4. A DNA fragment is "derived from" an ET-NANB viral agent if it has the same or substantially the same basepair sequence as a region of the viral agent genome.
5. A protein is "derived from" an ET-NANB viral agent if it is encoded by an open reading frame of a DNA or RNA fragment derived from an ET-NANB viral agent.

### II. Obtaining Cloned ET-NANB Fragments

It has been found that a virus-specific DNA clone can be produced by (a) isolating RNA from the bile of a cynomolgus monkey having a known ET-NANB infection, (b) cloning the cDNA fragments to form a fragment library, and (c) screening the library by differential hybridization to radiolabeled cDNAs from infected and non-infected bile sources.

### A. cDNA Fragment Mixture

ET-NANB infection in cynomolgus monkeys is initiated by inoculating the animals intravenously with a 10% w/v suspension from human case stools positive for 27-34 nm ET-NANB particles (mean diameter 32 nm). An infected animal is monitored for elevated levels of alanine aminotransferase, indicating hepatitis infection. ET-NANB infection is confirmed by immunospecific binding of seropositive antibodies to virus-like particles (VLPs), according to published methods (Gravelle). Briefly, a stool (or bile) specimen taken from the infected animal 3-4 weeks after infection is diluted 1:10 with phosphate-buffered saline, and the 10t suspension is clarified by low-speed centrifugation and filtration successively through 1.2 and 0.45 micron filters. The material may be further purified by pelleting through a 30% sucrose cushion (Bradley). The resulting preparation of VLPs is mixed with diluted serum from human patients with known ET-NANB infection. After incubation overnight, the mixture is centrifuged overnight to pellet immune aggregates, and these are stained and examined by electron microscopy for antibody binding to the VLPs.

ET-NANB infection can also be confirmed by seroconversion to VLP-positive serum. Here the serum of the infected animal is mixed as above with 27-34 nm VLPs isolated from the stool specimens of infected human cases and examined by immune electron microscopy for antibody binding to the VLPs.

Bile can be collected from ET-NANB positive animals by either cannulating the bile duct and collecting the bile fluid or by draining the bile duct during necropsy. Total RNA is extracted from the bile by hot phenol extraction, as outlined in Example 1A. The RNA fragments are used to synthesize corresponding duplex cDNA fragments by random priming, also as referenced in Example 1A. The cDNA fragments may be fractionated by gel electrophoresis or density gradient centrifugation to obtain a desired size class of fragments, e.g., 500-4,000 basepair fragments.

Although alternative sources of viral material, such as VLPs obtained from stool samples (as described in Example 4), may be used for producing a CDNA fraction, the bile source is preferred.

It has been found that bile from ET-NANB-infected monkeys shows a greater number of intact viral particles than material obtained from stool samples, as evidenced by immune electron microscopy. Bile obtained from an ET-NANB infected human or cynomolgus macaque, for use as a source of ET-NANB viral protein or genomic material, or intact virus, forms part of the present disclosure.

### B. cDNA Library and Screening

The cDNA fragments from above are cloned into a suitable cloning vector to form a cDNA library. This may be clone by equipping blunt-ended fragments with a suitable end linker, such as an EcoRI sequence, and inserting the fragments into a suitable insertion site of a cloning vector, such as at a unique EcoRI site. After initial cloning, the library may be re-cloned, if desired, to increase the percentage of vectors containing a fragment insert. The library construction described in Example 1B is illustrative. Here cDNA fragments were blunt-ended, equipped with EcoRI ends, and inserted into the EcoRI site of the lambda phage vector gt10. The library phage, which showed less than 5% fragment inserts, was isolated, and the fragment inserts re-cloned into the lambda gt10 vector, yielding more than 95% insert-containing phage.

The cDNA library is screened for sequences specific for ET-NANB by differential hybridization to cDNA probes derived from infected and non-infected sources, cDNA fragments from infected and non-infected source bile or stool viral isolates can be prepared as above. Radiolabeling the fragments is by random labeling, nick translation, or end labeling, according to conventional methods (Maniatis, p. 109). The cDNA library from above is screened by transfer to duplicate nitrocellulose filters, and hybridization with both infected-source and non-infected-source (control) radiolabeled probes, as detailed in Example 2. In order to recover sequences that hybridize at the preferred outer limit of 25-30% basepair mismatches, clones can be selected if they hybridize under the conditions described in Maniatis et al., op. cit., pp. 320-323, but using the following wash conditions: 2 x SCC, 0.1% SDS, room temperature - twice, 30 minutes each; then 2 x SCC, 0.1% SDS, 50°C - once, 30 minutes; then 2 x SCC, room temperature - twice, 10 minutes each. These conditions allowed identification of the Mexican isolate discussed above using the ET1.1 sequence as a probe. Plaques which show selective hybridization to the infected-source probes are preferably re-plated at low plating density and re-screened as above, to isolate single clones which are specific for ET-NANB sequences. As indicated in Example 2, sixteen clones which hybridized specifically with infected-source probes were identified by these procedures. One of the clones, designated lambda gt101.1, contained a 1.33 kilobase fragment insert.

### C. ET-NANB Sequences

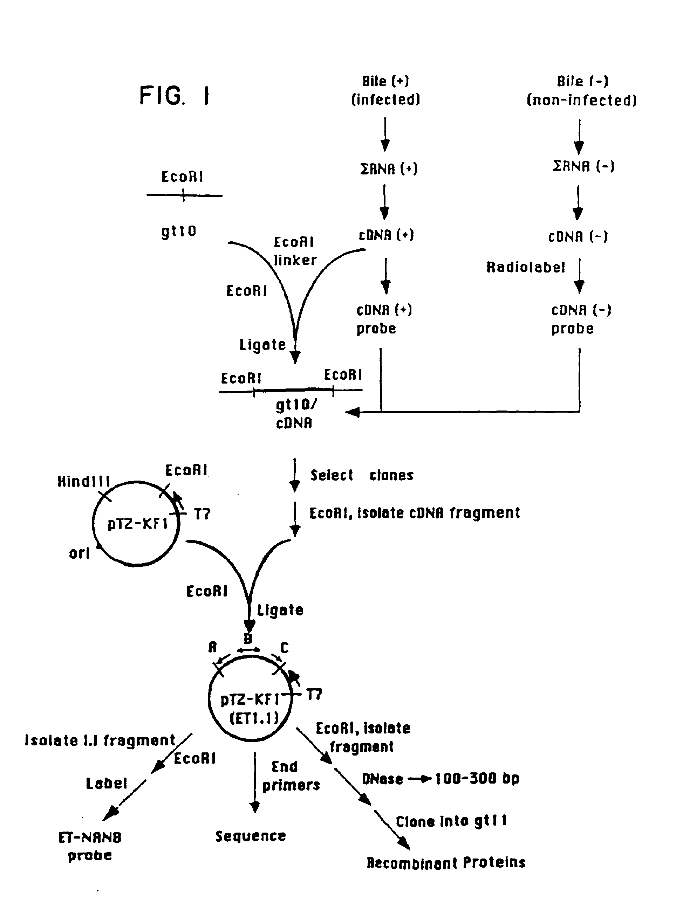

The basepair sequence of cloned regions of the ET-NANB fragments from Part B are determined by standard sequencing methods. In one illustrative method, described in Example 3, the fragment insert from the selected cloning vector is excised, isolated by gel electrophoresis, and inserted into a cloning vector whose basepair sequence on either side of the insertion site is known. The particular vector employed in Example 3 is a pTZKF1 vector shown at the left in Figure 1. The ET-NANB fragment from the gt10-1.1 phage was inserted at the unique EcoRI site of the pTZKF1 plasmid. Recombinants carrying the desired insert were identified by hybridization with the isolated 1.33 kilobase fragment, as described in Example 3. One selected plasmid, identified as pTZKF1 (ET1.1), gave the expected 1.33 kb fragment after vector digestion with EcoRI. E. coli strain BB4 infected with the pTZKF1(ET1.1) plasmid has been deposited with the American Type Culture Collection, Rockville, MD, and is identified by ATCC deposit number 67717.

The pTZKF1(ET1.1) plasmid is illustrated at the bottom in Figure 1. The fragment insert has 5' and 3' end regions denoted at A and C, respectively, and an intermediate region, denoted at B. The sequences in these regions were determined by standard dideoxy sequencing and were set forth in an earlier application in this series. The three short sequences (A, B, and C) are from the same insert strand. As will be seen in Example 3, the B-region sequence was actually determined from the opposite strand, so that the B region sequence shown above represents the complement of the sequence in the sequenced strand. The base numbers of the partial sequences are approximate.

Later work in the laboratory of the inventors identified the full sequence, set forth above. Fragments of this total sequence can readily be prepared using restriction endonucleases. Computer analysis of both the forward and reverse sequence has identified a number of cleavage sites.

### III. ET-NANB Fragments

According to another aspect, the invention includes ET-NANB-specific fragments or probes which hybridize with ET-NANB genomic sequences or cDNA fragments derived therefrom. The fragments may include full-length cDNA fragments such as described in Section II, or may be derived from shorter sequence regions within cloned cDNA fragments. Shorter fragments can be prepared by enzymatic digestion of full-length fragments under conditions which yield desired-sized fragments, as will be described in Section IV. Alternatively, the fragments can be produced by oligonucleotide synthetic methods, using sequences derived from the cDNA fragments. Methods or commercial services for producing selected-sequence oligonucleotide fragments are available. Fragments are usually at least 12 nucleotides in length, preferably at least 14, 20, 30 or 50 nucleotides, when used as probes. Probes can be full length or less than 500, preferably less than 300 or 200, nucleotides in length.

To confirm that a given ET-NANB fragment is in fact derived from the ET-NANB viral agent, the fragment can be shown to hybridize selectively with cDNA from infected sources. By way of illustration, to confirm that the 1.33 kb fragment in the pTZKF1(ET1.1) plasmid is ET-NANB in origin, the fragment was excised from the pTZKF1(ET1.1) plasmid, purified, and radiolabeled by random labeling. The radiolabeled fragment was hybridized with fractionated cDNAs from infected and non-infected sources to confirm that the probe reacts only with infected-source cDNAs. This method is illustrated in Example 4, where the above radiolabeled 1.33 kb fragment from pTZKFl(ET1.1) plasmid was examined for binding to cDNAs prepared from infected and non-infected sources. The infected sources are (1) bile from a cynomolgus macaque infected with a strain of virus derived from stool samples from human patients from Burma with known ET-NANB infections and (2) a viral agent derived from the stool sample of a human ET-NANB patient from Mexico. The cDNAs in each fragment mixture were first amplified by a linker/primer amplification method described in Example 4. Fragment separation was on agarose gel, followed by Southern blotting and then hybridization to bind the radiolabeled 1.33 kb fragment to the fractionated cDNAs. The lane containing cDNAs from the infected sources showed a smeared band of bound probe, as expected (cDNAs amplified by the linker/primer amplification method would be expected to have a broad range of sizes). No probe binding to the amplified cDNAs from the non-infected sources was observed. The results indicate that the 1.33 kb probe is specific for cDNA fragments associated with ET-NANB infection. This same type of study, using ET 1.1 as the probe, has demonstrated hybridization to ET-NANB samples collected from Tashkent, Somalia, Borneo and Pakistan. Secondly, the fact that the probe is specific for ET-NANB related sequences derived from different continents (Asia, Africa and North America) indicates the cloned ET-NANB Burma sequence (ET1.1) is derived from a common ET-NANB virus or virus class responsible for ET-NANB hepatitis infection worldwide.

In a related confirmatory study, probe binding to fractionated genomic fragments prepared from human or cynomolgus macaque genomic DNA (both infected and uninfected) was examined. No probe binding was observed to either genomic fraction, demonstrating that the ET-NANB fragment is not an endogenous human or cynomolgus genomic fragment and additionally demonstrating that HEV is an RNA virus.

Another confirmation of ET-NANB specific sequences in the fragments is the ability to express ET-NANB proteins from coding regions in the fragments and to demonstrated specific sero-reactivity of these proteins with .sera collected during documented outbreaks of ET-NANB. Section IV below discusses methods of protein expression using the fragments.

One important use of the ET-NANB-specific fragments is for identifying ET-NANB-derived cDNAs which contain additional sequence information. The newly identified cDNAs, in turn, yield new fragment probes, allowing further iterations until the entire viral genome is identified and sequenced. Procedures for identifying additional ET-NANB library clones and generating new probes therefrom generally follow the cloning and selection procedures described in Section II.

The fragments (and oligonucleotides prepared based on the sequences given above) are also useful as primers for a polymerase chain reaction method of detecting ET-NANB viral genomic material in a patient sample. This diagnostic method will be described in Section V below.

Two specific genetic sequences derived from the Mexican strain, identified herein as 406.3-2 and 406.4-2, have been identified that encode immunogenic epitopes. This was clone by isolating clones which encode epitopes that immunologically react specifically with sera from individuals and experimental animals infected with HEV. Comparison of the isolated sequences with those in the Genebank collection of genetic sequences indicate that these viral sequences are novel. Since these sequences are unique, they can be used to identify the presence of HEV and to distinguish this strain of hepatitis from HAV, HBV, and HCV strains. The sequences are also useful for the design of oligonucleotide probes to diagnose the presence of virus in samples. They can be used for the synthesis of polypeptides that themselves are used in immunoassays. The specific 406.3-2 and 406.4-2 sequences can be incorporated into other genetic material, such as vectors, for ease of expression or replication. They can also be used (as demonstrated above) for identifying similar antigenic regions encoded by related viral strains, such as the Burmese strain.

### IV. ET-NANB Proteins

As indicated above, ET-NANB proteins can be prepared by expressing open reading-frame coding regions in ET-NANB fragments. In one preferred approach, the ET-NANB fragments used for protein expression are derived from cloned cDNAs which have been treated to produce desired-size fragments, and preferably random fragments with sizes predominantly between about 100 to about 300 base pairs. Example 5 describes the preparation of such fragments by DNAs digestion. Because it is desired to obtain peptide antigens of between about 30 to about 100 amino acids, the digest fragments are preferably size fractionated, for example by gel electrophoresis, to select those in the approximately 100-300 basepair size range. Alternatively, cDNA libraries constructed directly from HEV-containing sources (e.g., bile or stool) can be screened directly if cloned into an appropriate expression vector (see below).

For example, the ET-NANB proteins expressed by the 406.3-2 and 406.4-2 sequences (and peptide fragments thereof) are particularly preferred since these proteins have been demonstrated to be immunoreactive with a variety of different human sera, thereby indicating the presence of one or more epitopes specific for HEV on their surfaces. These clones were identified by direct screening of a gt11 library.

### A. Expression Vector

The ET-NANB fragments are inserted into a suitable expression vector. One exemplary expression vector is lambda gt11, which contains a unique EcoRI insertion site 53 base pairs upstream of the translation termination codon of the beta-galactosidase gene. Thus, the inserted sequence will be expressed as a beta-galactosidase fusion protein which contains the N-terminal portion of the beta-galactosidase gene, the heterologous peptide, and optionally the C-terminal region of the beta-galactosidase peptide (the C-terminal portion being expressed when the heterologous peptide coding sequence does not contain a translation termination codon). This vector also produces a temperature-sensitive repressor (c1857) which causes viral lysogeny at permissive temperatures, e.g., 32°C, and leads to viral lysis at elevated temperatures, e.g., 37°C. Advantages of this vector include: (1) highly efficient recombinant generation, (2) ability to select lysogenized host cells on the basis of host-cell growth at permissive, but not non-permissive, temperatures, and (3) high levels of recombinant fusion protein production. Further, since phage containing a heterologous insert produces an inactive beta-galactosidase enzyme, phage with inserts can be readily identified by a beta-galactosidase colored-substrate reaction.

For insertion into the expression vector, the viral digest fragments may be modified, if needed, to contain selected restriction-site linkers, such as EcoRI linkers, according to conventional procedures. Example 1 illustrates methods for cloning the digest fragments into lambda gt11, which includes the steps of blunt-ending the fragments, ligating with EcoRI linkers, and introducing the fragments into EcoRI-cut lambda gt11. The resulting viral genomic library may be checked to confirm that a relatively large (representative) library has been produced. This can be done, in the case of the lambda gt11 vector, by infecting a suitable bacterial host, plating the bacteria, and examining the plaques for loss of beta-galactosidase activity. Using the procedures described in Example 1, about 50% of the plaques showed loss of enzyme activity.

### B. Peptide Antigen Expression

The viral genomic library formed above is screened for production of peptide antigen (expressed as a fusion protein) which is immunoreactive with antiserum from ET-NANB seropositive individuals . In a preferred screening method, host cells infected with phage library vectors are plated, as above, and the plate is blotted with a nitrocellulose filter to transfer recombinant protein antigens produced by the cells onto the filter. The filter is then reacted with the ET-NANB antiserum, washed to remove unbound antibody, and reacted with reporter-labeled, anti-human antibody, which becomes bound to the filter, in sandwich fashion, through the anti-ET-NANB antibody.

Typically phage plaques which are identified by virtue of their production of recombinant antigen of interest are re-examined at a relatively low density for production of antibody-reactive fusion protein. Several recombinant phage clones which produced immunoreactive recombinant antigen were identified in the procedure.

The selected expression vectors may be used for scale-up production, for purposes of recombinant protein purification. Scale-up production is carried out using one of a variety of reported methods for (a) lysogenizing a suitable host, such as E. coli, with a selected lambda gt11 recombinant (b) culturing the transduced cells under conditions that yield high levels of the heterologous peptide, and (c) purifying the recombinant antigen from the lysed cells.

In one preferred method involving the above lambda gt11 cloning vector, a high-producer E. coli host, BNN103, is infected with the selected library phage and replica plated on two plates. One of the plates is grown at 32°C, at which viral lysogeny can occur, and the other at 42°C, at which the infecting phage is in a lytic stage and therefore prevents cell growth. Cells which grow at the lower but not the higher temperature are therefore assumed to be successfully lysogenized.

The lysogenized host cells are then grown under liquid culture conditions which favor high production of the fused protein containing the viral insert, and lysed by rapid freezing to release the desired fusion protein.

### C. Peptide Purification

The recombinant peptide can be purified by standard protein purification procedures which may include differential precipitation, molecular sieve chromatography, ion-exchange chromatography, isoelectric focusing, gel electrophoresis and affinity chromatography. In the case of a fused protein, such as the beta-galactosidase fused protein prepared as above, the protein isolation techniques which are used can be adapted from those used in isolation of the native protein. Thus, for isolation of a soluble betagalactosidase fusion protein, the protein can be isolated readily by simple affinity chromatography, by passing the cell lysis material over a solid support having surface-bound anti-beta-galactosidase antibody.

### D. Viral Proteins

The ET-NANB protein of the invention may also be derived directly from the ET-NANB viral agent. VLPs or protein isolated from stool or liver samples from an infected individual, as above, are one suitable source of viral protein material. The VLPs isolated from the stool sample may be further purified by affinity chromatography prior to protein isolation (see below). The viral agent may also be raised in cell culture, which provides a convenient and potentially concentrated source of viral protein. Co-owned U.S. Patent Application Serial No. 846,757, filed April 1, 1986, describes an immortalized trioma liver cell which supports NANB infection in cell culture. The trioma cell line is prepared by fusing human liver cells with a mouse/human fusion partner selected for human chromosome stability. Cells containing the desired NANB viral agent can be identified by immunofluorescence methods, employing anti-ET-NANB human antibodies.

The viral agent is disrupted, prior to protein isolation, by conventional methods, which can include sonication, high- or low-salt conditions, or use of detergents.

Purification of ET-NANB viral protein can be carried out by affinity chromatography, using a purified anti-ET-NANB antibody attached according to standard methods to a suitable solid support. The antibody itself may be purified by affinity chromatography, where an immunoreactive recombinant ETNANB protein, such as described above, is attached to a solid support, for isolation of anti-ET-NANB antibodies from an immune serum source. The bound antibody is released from the support by standard methods.

Alternatively, the anti-ET-NANB antibody may be an antiserum or a monoclonal antibody (Mab) prepared by immunizing a mouse or other animal with recombinant ETNANB protein. For Mab production, lymphocytes are isolated from the animal and immortalized with a suitable fusion partner, and successful fusion products which react with the recombinant protein immunogen are selected. These in turn may be used in affinity purification procedures, described above, to obtain native ET-NANB antigen.

### V. Utility

Although ET-NANB is primarily of interest because of its effects on humans, recent data has shown that this virus is also capable of infecting other animals, especially mammals. Accordingly, any discussion herein of utility applies to both human and veterinary uses, especially commercial veterinary uses, such as the diagnosis and treatment of pigs, cattle, sheep, horses, and other domesticated animals.

### A. Diagnostic Methods

The particles and antigens disclosed herein, as well as the genetic material, can be used in diagnostic assays. Methods for detecting the presence of ET-NANB hepatitis comprise analyzing a biological sample such as a blood sample, stool sample or liver biopsy specimen for the presence of an analyte associated with ET-NANB hepatitis virus.

The analyte can be a nucleotide sequence which hybridizes with a probe comprising a sequence of at least about 16 consecutive nucleotides, usually 30 to 200 nucleotides, up to substantially the full sequence of the sequences shown above (cDNA sequences). The analyte can be RNA or cDNA. The analyte is typically a virus particle suspected of being ET-NANB or a particle for which this classification is being ruled out. The virus particle can be further characterized as having an RNA viral genome comprising a sequence at least about 70% homologous to a sequence of at least 12 consecutive nucleotides of the "forward" and "reverse" sequences given above, usually at least about 80% homologous to at least about 60 consecutive nucleotides within the sequences, and may comprise a sequence substantially homologous to the full-length sequences. In order to detect an analyte, where the analyte hybridizes to a probe, the probe may contain a detectable label. Particularly preferred for use as a probe are sequences of consecutive nucleotides derived from the 406.3-2 and 406.4-2 clones described herein, since these clones appear to be particularly diagnostic for HEV.

The analyte can also comprise an antibody which recognizes an antigen, such as a cell surface antigen, on a ET-NANB virus particle. The analyte can also be a ET-NANB viral antigen. Where the analyte is an antibody or an antigen, either a labelled antigen or antibody, respectively, can be used to bind to the analyte to form an immunological complex, which can then be detected by means of the label.

Typically, methods for detecting analytes such as surface antigens and/or whole particles are based on immunoassays. Immunoassays can be conducted either to determine the presence of antibodies in the host that have arisen from infection by ET-NANB hepatitis virus or by assays that directly determine the presence of virus particles or antigens. Such techniques are well known and need not be described here in detail. Examples include both heterogeneous and homogeneous immunoassay techniques. Both techniques are based on the formation of an immunological complex between the virus particle or its antigen and a corresponding specific antibody. Heterogeneous assays for viral antigens typically use a specific monoclonal or polyclonal antibody bound to a solid surface. Sandwich assays are becoming increasingly popular. Homogeneous assays, which are carried out in solution without the presence of a solid phase, can also be used, for example by determining the difference in enzyme activity brought on by binding of free antibody to an enzyme-antigen conjugate. A number of suitable assays are disclosed in U.S. Patent Nos. 3,817,837, 4,006,360, 3,996,345.

When assaying for the presence of antibodies induced by ET-NANB viruses, the viruses and antigens described herein can be used as specific binding agents to detect either IgG or IgM antibodies. Since IgM antibodies are typically the first antibodies that appear during the course of an infection, when IgG synthesis may not yet have been initiated, specifically distinguishing between IgM and IgG antibodies present in the blood stream of a host will enable a physician or other investigator to determine whether the infection is recent or convalescent. Proteins expressed by the 406.3-2 and 406.4-2 clones described herein and peptide fragments thereof are particularly preferred for use as specific binding agents to detect antibodies since they have been demonstrated to be reactive with a number of different human HEV sera. Further, they are reactive with both acute and convalescent sera.

In one diagnostic configuration, test serum is reacted with a solid phase reagent having surface-bound ET-NANB protein antigen. After binding anti-ET-NANB antibody to the reagent and removing unbound serum components by washing, the reagent is reacted with reporter-labeled anti-human antibody to bind reporter to the reagent in proportion to the amount of bound anti-ET-NANB antibody on the solid support. The reagent is again washed to remove unbound labeled antibody, and the amount of reporter associated with the reagent is determined. Typically, the reporter is an enzyme which is detected by incubating the solid phase in the presence of a suitable fluorometric or colorimetric substrate.

The solid surface reagent in the above assay prepared by known techniques for attaching protein material to solid support material, such as polymeric beads, dip sticks, or filter material. These attachment methods generally include non-specific adsorption of the protein to the support or covalent attachment of the protein, typically through a free amine group, to a chemically reactive group on the solid support, such as an activate carboxyl, hydroxyl, or aldehyde group.

In a second diagnostic configuration, known as a homogeneous assay, antibody binding to a solid support produces some change in the reaction medium which can be directly detected in the medium. Known general types of homogeneous assays proposed heretofore include (a) spin-labeled reporters, where antibody binding to the antigen is detected by a change in reported mobility (broadening of the spin splitting peaks), (b) fluorescent reporters, where binding is detected by a change in fluorescence efficiency, (c) enzyme reporters, where antibody binding effects enzyme/substrate interactions, and (d) liposome-bound reporters, where binding leads to liposome lysis and release of encapsulated reporter. The adaptation of these methods to the protein antigen of the present invention follows conventional methods for preparing homogeneous assay reagents.

In each of the assays described above, the assay method involves reacting the serum from a test individual with the protein antigen and examining the antigen for the presence of bound antibody. The examining may involve attaching a labeled anti-human antibody to the antibody being examined, either IgM (acute phase) or IgG (convalescent phase), and measuring the amount of reporter bound to the solid support, as in the first method, or may involve observing the effect of antibody binding on a homogeneous assay reagent, as in the second method.

Also described herein is an assay system or kit for carrying out the assay method just described. The kit generally includes a support with surface-bound recombinant protein antigen which is (a) immunoreactive with antibodies present in individuals infected with enterically transmitted nonA/nonB viral agent and (b) derived from a viral hepatitis agent whose genome contains a region which is homologous to the 1.33 kb DNA EcoRI insert present in plasmid pTZKF1(ET1.1) carried in E. Coli strain BB4, and having ATCC deposit no. 67717. A reporter-labeled anti-human antibody in the kit is used for detecting surface-bound anti-ET-NANB antibody.

### B. Viral Genome Diagnostic Applications

The genetic material of the invention can itself be used in numerous assays as probes for genetic material present in naturally occurring infections. One method for amplification of target nucleic acids, for later analysis by hybridization assays, is known as the polymerase chain reaction or PCR technique. The PCR technique can be applied to detecting virus particles as described herein in suspected pathological samples using oligonucleotide primers spaced apart from each other and based on the genetic sequence set forth above. The primers are complementary to opposite strands of a double stranded DNA molecule and are typically separated by from about 50 to 450 nt or more (usually not more than 2000 nt). This method entails preparing the specific oligonucleotide primers and then repeated cycles of target DNA denaturation, primer binding, and extension with a DNA polymerase to obtain DNA fragments of the expected length based on the primer spacing. Extension products generated from one primer serve as additional target sequences for the other primer. The degree of amplification of a target sequence is controlled by the number of cycles that are performed and is theoretically calculated by the simple formula 2ⁿ where n is the number of cycles. Given that the average efficiency per cycle ranges from about 65% to 85%, 25 cycles produce from 0.3 to 4.8 million copies of the target sequence. The PCR method is described in a number of publications, including Saiki et al., Science (1985) 230:1350-1354; Saiki et al., Nature (1986) 324:163-166; and Scharf et al., Science (1986) 233:1076-1078. Also see U.S. Patent Nos. 4,683,194; 4,683,195; and 4,683,202.

The invention includes a specific diagnostic method for determination of ET-NANB viral agent, based on selective amplification of ET-NANB fragments. This method employs a pair of single-strand primers derived from non-homologous regions of opposite strands of a DNA duplex fragment, which in turn is derived from an enterically transmitted viral hepatitis agent whose genome contains a region which is homologous to the 1.33 kb DNA EcoRI insert present in plasmid pTZKF1(ET1.1) carried in E. coli strain BB4, and having ATCC deposit no. 67717. These "primer fragments" are prepared from ET-NANB fragments such as described in Section III above. The method follows the process for amplifying selected nucleic acid sequences as disclosed in U.S. Patent No. 4,683,202, as discussed above.

### C. Peptide Vaccine

Any of the antigens of the invention can be used in preparation of a vaccine. A preferred starting material for preparation of a vaccine is the particle antigen isolated from bile. The antigens are preferably initially recovered as intact particles as described above. However, it is also possible to prepare a suitable vaccine from particles isolated from other sources or non-particle recombinant antigens. When non-particle antigens are used (typically soluble antigens), proteins derived from the viral envelope or viral capsid are preferred for use in preparing vaccines. These proteins can be purified by affinity chromatography, also described above.

If the purified protein is not immunogenic per se, it can be bound to a carrier to make the protein immunogenic. Carriers include bovine serum albumin, keyhole limpet hemocyanin and the like. It is desirable, but not necessary, to purify antigens to be substantially free of human protein. However, it is more important that the antigens be free of proteins, viruses, and other substances not of human origin that may have been introduced by way of, or contamination of, the nutrient medium, cell lines, tissues, or pathological fluids from which the virus is cultured or obtained.

Vaccination can be conducted in conventional fashion. For example, the antigen, whether a viral particle or a protein, can be used in a suitable diluent such as water, saline, buffered salines, complete or incomplete adjuvants, and the like. The immunogen is administered using standard techniques for antibody induction, such as by subcutaneous administration of physiologically compatible, sterile solutions containing inactivated or attenuated virus particles or antigens. An immune response producing amount of virus particles is typically administered per vaccinizing injection, typically in a volume of one milliliter or less.

A specific example of a vaccine composition includes, in a pharmacologically acceptable adjuvant, a recombinant protein or protein mixture derived from an enterically transmitted nonA/nonB viral hepatitis agent whose genome contains a region which is homologous to the 1.33 kb DNA EcoRI insert present in plasmid pTZKF1(ETI.1) carried in E. coli strain BB4, and having ATCC deposit no. 67717. The vaccine is administered at periodic intervals until a significant titer of anti-ET-NANB antibody is detected in the serum. The vaccine is intended to protect against ET-NANB infection.

Particularly preferred are vaccines prepared using proteins expressed by the 406.3-2 and 406.4-2 clones described herein and equivalents thereof, including fragments of the expressed proteins. Since these clones have already been demonstrated to be reactive with a variety of human HEV-positive sera, their utility in protecting against a variety of HEV strains is indicated.

### D. Prophylactic and Therapeutic

### Antibodies and Antisera

In addition to use as a vaccine, the compositions can be used to prepare antibodies to ET-NANB virus particles. The antibodies can be used directly as antiviral agents. To prepare antibodies, a host animal is immunized using the virus particles or, as appropriate, non-particle antigens native to the virus particle are bound to a carrier as described above for vaccines. The host serum or plasma is collected following an appropriate time interval to provide a composition comprising antibodies reactive with the virus particle. The gamma globulin fraction or the IgG antibodies can be obtained, for example, by use of saturated ammonium sulfate or DEAE Sephadex, or other techniques known to those skilled in the art. The antibodies are substantially free of many of the adverse side effects which may be associated with other anti-viral agents such as drugs.

The antibody compositions can be made even more compatible with the host system by minimizing potential adverse immune system responses. This is accomplished by removing all or a portion of the FC portion of a foreign species antibody or using an antibody of the same species as the host animal, for example, the use of antibodies from human/human hybridomas.

The antibodies can also be used as a means of enhancing the immune response since antibody-virus complexes are recognized by macrophages. The antibodies can be administered in amounts similar to those used for other therapeutic administrations of antibody. For example, pooled gamma globulin is administered at 0.02-0.1 ml/lb body weight during the early incubation of other viral diseases such as rabies, measles and hepatitis B to interfere with viral entry into cells. Thus, antibodies reactive with the ET-NANB virus particle can be passively administered alone or in conjunction with another anti-viral agent to a host infected with an ET-NANB virus to enhance the immune response and/or the effectiveness of an antiviral drug.

Alternatively, anti-ET-NANB-virus antibodies can be induced by administering anti-idiotype antibodies as immunogens. Conveniently, a purified antiET-NANB-virus antibody preparation prepared as described above is used to induce anti-idiotype antibody in a host animal. The composition is administered to the host animal in a suitable diluent. Following administration, usually repeated administration, the host produces anti-idiotype antibody. To eliminate an immunogenic response to the Fc region, antibodies produced by the same species as the host animal can be used or the Fc region of the administered antibodies can be removed. Following induction of anti-idiotype antibody in the host animal, serum or plasma is removed to provide an antibody composition. The composition can be purified as described above for anti-ET-NANB virus antibodies, or by affinity chromatography using anti-ET-NANB-virus antibodies bound to the affinity matrix. The anti-idiotype antibodies produced are similar in conformation to the authentic ET-NANB antigen and may be used to prepare an ET-NANB vaccine rather than using a ET-NANB particle antigen.

When used as a means of inducing anti-ET-NANB virus antibodies in a patient, the manner of injecting the antibody is the same as for vaccination purposes, namely intramuscularly, intraperitoneally, subcutaneously or the like in an effective concentration in a physiologically suitable diluent with or without adjuvant. One or more booster injections may be desirable. The anti-idiotype method of induction of anti-ET-NANB virus antibodies can alleviate problems which may be caused by passive administration of anti-ET-NANB-virus antibodies, such as an adverse immune response, and those associated with administration of purified blood components, such as infection with as yet undiscovered viruses.

The ET-NANB derived proteins of the invention are also intended for use in producing antiserum designed for pre- or post-exposure prophylaxis. Here an ET-NANB protein, or mixture of proteins is formulated with a suitable adjuvant and administered by injection to human volunteers, according to known methods for producing human antisera. Antibody response to the injected proteins is monitored, during a several- week period following immunization, by periodic serum sampling to detect the presence an anti-ET-NANB serum antibodies, as described in Section IIA above.

The antiserum from immunized individuals may be administered as a pre-exposure prophylactic measure for individuals who are at risk of contracting infection. The antiserum is also useful in treating an individual post-exposure, analogous to the use of high titer antiserum against hepatitis B virus for post-exposure prophylaxis.

### E. Monoclonal Antibodies

For both in vivo use of antibodies to ET-NANB virus particles and proteins and anti-idiotype antibodies and diagnostic use, it may be preferable to use monoclonal antibodies. Monoclonal anti-virus particle antibodies or anti-idiotype antibodies can be produced as follows. The spleen or lymphocytes from an immunized animal are removed and immortalized or used to prepare hybridomas by methods known to those skilled in the art. To produce a human-human hybridoma, a human lymphocyte donor is selected. A donor known to be infected with a ET-NANB virus (where infection has been shown for example by the presence of anti-virus antibodies in the blood or by virus culture) may serve as a suitable lymphocyte donor. Lymphocytes can be isolated from a peripheral blood sample or spleen cells may be used if the donor is subject to splenectomy. Epstein-Barr virus (EBV) can be used to immortalize human lymphocytes or a human fusion partner can be used to produce human-human hybridomas. Primary in vitro immunization with peptides can also be used in the generation of human monoclonal antibodies.

Antibodies secreted by the immortalized cells are screened to determine the clones that secrete antibodies of the desired specificity. For monoclonal anti-virus particle antibodies, the antibodies must bind to ET-NANB virus particles. For monoclonal anti-idiotype antibodies, the antibodies must bind to anti-virus particle antibodies. Cells producing antibodies of the desired specificity are selected.

The following examples illustrate various aspects of the invention, but are in no way intended to limit the scope thereof.

### Material

The materials used in the following Examples were as follows:

Enzymes: DNAse I and alkaline phosphatase were obtained from Boehringer Mannheim Biochemicals (BMB, Indianapolis, IN); EcoRI, EcoRI methylase, DNA ligase, and DNA Polymerase I, from New England Biolabs (NEB, Beverly MA); and RNase A was obtained from Sigma (St. Louis, MO) .

Other reagents: EcoRI linkers were obtained from NEB; and nitro blue tetrazolium (NBT), S-bromo-4-chloro-3-indolyl phosphate (BCIP) S-bromo-4-chloro-3-indolyl-B-D-galactopyranoside (Xgal) and isopropyl B-D-thiogalactopyranoside (IPTG) were obtained from Sigma.

cDNA synthesis kit and random priming labeling kits are available from Boehringer-Mannheim Biochemical (BMB, Indianapolis, IN).

### Example 1

### Preparing cDNA Library

### A. Source of ET-NANB virus

Two cynomolgus monkeys (cynos) were intravenously injected with a 10% suspension of a stool pool obtained from a second-passage cyno (cyno #37) infected with a strain of ET-NANB virus isolated from Burma cases whose stools were positive for ET-NANB, as evidenced by binding of 27-34 nm virus-like particles (VLPs) in the stool to immune serum from a known ETNANB patient. The animals developed elevated levels of alanine aminotransferase (ALT) between 24-36 days after inoculation, and one excreted 27-34 nm VLPs in its bile in the pre-acute phase of infection.

The bile duct of each infected animal was cannulated and about 1-3 cc of bile was collected daily. RNA was extracted from one bile specimen (cyno #121) by hot phenol extraction, using a standard RNA isolation procedure. Double-strand cDNA was formed from the isolated RNA by a random primer for first-strand generation, using a cDNA synthesis kit obtained from Boehringer-Mannheim (Indianapolis, IN).

### B. Cloning the Duplex Fragments

The duplex cDNA fragments were blunt-ended with T4 DNA polymerase under standard conditions (Maniatis, p. 118), then extracted with phenol/chloroform and precipitated with ethanol. The blunt-ended material was ligated with EcoRI linkers under standard conditions (Maniatis, pp. 396-397) and digested with EcoRI to remove redundant linker ends. Non-ligated linkers were removed by sequential isopropanol precipitation.

Lambda gt10 phage vector (Huynh) was obtained from Promega Biotec (Madison, WI). This cloning vector has a unique EcoRI cloning site in the phage CI repressor gene. The cDNA fragments from above were introduced into the EcoRI site by mixing 0.5 - 1.0 µg EcoRI-cleaved gt10, 0.5-3 µl of the above duplex fragments, 0.5 µl 10X ligation buffer, 0.5 µl ligase (200 units), and distilled water to 5 µl. The mixture was incubated overnight at 14°C, followed by in vitro packaging, according to standard methods (Maniatis, pp. 256-268).

The packaged phage were used to infect an E. coli hfl strain, such as strain HG415. Alternatively, E. coli, strain C600 hfl available from Promega Biotec, Madison, WI, could be used. The percentage of recombinant plaques obtained with insertion of the EcoRI-ended fragments was less than 5% by analysis of 20 random plaques.

The resultant cDNA library was plated and phage were eluted from the selection plates by addition of elution buffer. After DNA extraction from the phage, the DNA was digested with EcoRI to release the heterogeneous insert population, and the DNA fragments were fractionated on agarose to remove phage fragments. The 500-4,000 basepair inserts were isolated and recloned into lambda gt10 as above, and the packaged phage was used to infect E. coli strain HG415. The percentage of successful recombinants was greater than 95%. The phage library was plated on E. coli strain HG415, at about 5,000 plaques/plate, on a total of 8 plates.

### Example 2

### Selecting ET-NANB Cloned Fragments

### A. cDNA Probes

Duplex cDNA fragments from noninfected and ETNANB-infected cynomolgus monkeys were prepared as in Example 1. The cDNA fragments were radiolabeled by random priming, using a random-priming labeling kit obtained from Boehringer-Mannheim (Indianapolis, IN).

### B. Clone Selection

The plated cDNA library from Example 1 was transferred to each of two nitrocellulose filters, and the phage DNA was fixed on the filters by baking, according to standard methods (Maniatis, pp. 320323). The duplicate filters were hybridized with either infected-source or control CDNA probes from above. Autoradiographs of the filters were examined to identify library clones which hybridized with radiolabeled CDNA probes from infected source only, i.e., did not hybridize with cDNA probes from the non-infected source. Sixteen such clones, out of a total of about 40,000 clones examined, were identified by this subtraction selection method.

Each of the sixteen clones was picked and replated at low concentration on an agar plate. The clones on each plate were transferred to two nitrocellulose ag duplicate lifts, and examined for hybridization to radiolabeled cDNA probes from infected and noninfected sources, as above. Clones were selected which showed selective binding for infected-source probes (i.e., binding with infected-source probes and substantially no binding with non-infected-source probes). One of the clones which bound selectively to probe from infected source was isolated for further study. The selected vector was identified as lambda gt1O-1.1, indicated in Figure 1.

### Example 3

### ET-NANB Sequence

Clone lambda gt10-1.1 from Example 2 was digested with EcoRI to release the heterologous insert, which was separated from the vector fragments by gel electrophoresis. The electrophoretic mobility of the fragment was consistent with a 1.33 kb fragment. This fragment, which contained EcoRI ends, was inserted into the EcoRI site of a pTZKF1 vector, whose construction and properties are described in co-owned U.S. patent application for "Cloning Vector System and Method for Rare Clone Identification", Serial No. 125, 650, filed November 25, 1987. Briefly, and as illustrated in Figure 1, this plasmid contains a unique EcoRI site adjacent a T7 polymerase promoter site, and plasmid and phage origins of replication. The sequence immediately adjacent each side of the EcoRI site is known. E. coli BB4 bacteria, obtained from Stratagene (La Jolla, CA, were transformed with the plasmid.

Radiolabeled ET-NANB probe was prepared by excising the 1.33 kb insert from the lambda gt10-1.1 phage in Example 2, separating the fragment by gel electrophoresis, and randomly labeling as above. Bacteria transfected with the above pTZKF1 and containing the desired ET-NANB insert were selected by replica lift and hybridization with the radiolabeled ET-NANB probe, according to methods outlined in Example 2.

One bacterial colony containing a successful recombinant was used for sequencing a portion of the 1.33 kb insert. This isolate, designated pTZKF1(ET1.1), has been deposited with the American Type Culture Collection, and is identified by ATCC deposit no. 67717. Using a standard dideoxy sequencing procedure, and primers for the sequences flanking the EcoRI site, about 200-250 basepairs of sequence from the 5'-end region and 3'-end region of the insert were obtained. The sequences are given above in Section II. Later sequencing by the same techniques gave the full sequence in both directions, also given above.

### Example 4

### Detecting ET-NANB Sequences

cDNA fragment mixtures from the bile of noninfected and ET-NANB-infected cynomolgus monkeys were prepared as above. The cDNA fragments obtained from human stool samples were prepared as follows. Thirty ml of a 10% stool suspension obtained from an individual from Mexico diagnosed as infected with ET-NANB as a result of an ET-NANB outbreak, and a similar volume of stool from a healthy, non-infected individual, were layered over a 30% sucrose density gradient cushion, and centrifuged at 25,000 x g for 6 hr in an SW27 rotor, at 15°C. The pelleted material from the infected-source stool contained 27-34 nm VLP particles characteristic of ET-NANB infection in the infected-stool sample. RNA was isolated from the sucrose-gradient pellets in both the infected and non-infected samples, and the isolated RNA was used to produce cDNA fragments as described in Example 1.

The CDNA fragment mixtures from infected and non-infected bile source, and from infected and non-infected human-stool source were each amplified by a novel linker/primer replication method described in co-owned patent application serial number 07/208,512 for "DNA Amplification and Subtraction Technique," filed June 17, 1988. Briefly, the fragments in each sample were blunt-ended with DNA Pol I then extracted with phenol/chloroform and precipitated with ethanol. The blunt-ended material was ligated with linkers having the following sequence (top or 5' sequence has SEQ ID NO.21; bottom or 3'sequence has SEQ ID NO:22):
5'-GGAATTCGCGGCCGCTCG-3'
3'-TTCCTTAAGCGCCGGCGAGC-5'

The duplex fragments were digested with NruI to remove linker dimers, mixed with a primer having the sequence 5'-GGAATTCGCGGCCGCTCG-3', and then heat denatured and cooled to room temperature to form single-strand DNA/primer complexes. The complexes were replicated to form duplex fragments by addition of Thermus aquaticus (Taq) polymerase and all four deoxynucleotides. The replication procedures, involving successive strand denaturation, formation of strand/primer complexes, and replication, was repeated 25 times.

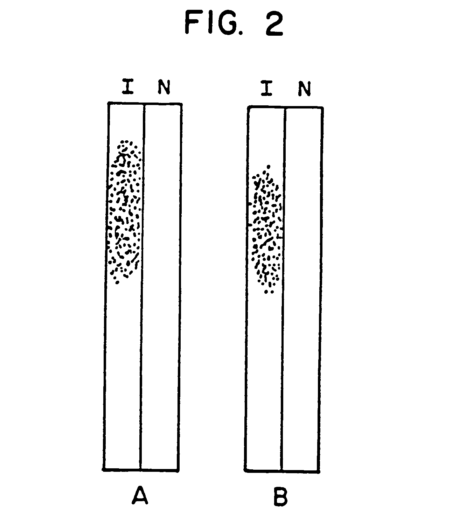

The amplified cDNA sequences were fractionated by agarose gel electrophoresis, using a 2% agarose matrix. After transfer of the DNA fragments from the agarose gels to nitrocellulose paper, the filters were hybridized to a random-labeled 32p probe prepared by (i) treating the pTZKF1(ET1.1) plasmid from above with EcoRI, (ii) isolating the released 1.33 kb ET-NANB fragment, and (iii) randomly labeling the isolated fragment. The probe hybridization wag performed by conventional Southern blotting methods (Maniatis, pp. 382-389). Figure 2 shows the hybridization pattern obtained with cDNAs from infected (I) and non-infected (N) bile sources (2A) and from infected (I) and noninfected (N) human stool sources (2B). As seen, the ET-NANB probe hybridized with fragments obtained from both of the infected sources, but was non-homologous to sequences obtained from either of the non-infected sources, thus confirming the specificity of derived sequence.

Southern blots of the radiolabeled 1.33 kb fragment with genomic DNA fragments from both human and cynomolgus-monkey DNA were also prepared. No probe hybridization to either of the genomic fragment mixtures was observed, confirming that the ET-NANB sequence is exogenous to either human or cynomolgus genome.

### Example 5

### Expressing ET-NANB Proteins

### A. Preparing ET-NANB Coding Sequences

The pTZKF1(ET1.1) plasmid from Example 2 was digested with EcoRI to release the 1.33 kb ET-NANB insert which was purified from the linearized plasmid by gel electrophoresis. The purified fragment was suspended in a standard digest buffer (0.5M Tris HCl, pH 7.5; 1 mg/ml BSA; 10mM MnC12) to a concentration of about 1 mg/ml and digested with DNAse I at room temperature for about 5 minutes. These reaction conditions were determined from a prior calibration study, in which the incubation time required to produce predominantly 100-300 basepair fragments was determined. The material was extracted with phenol/chloroform before ethanol precipitation.

The fragments in the digest mixture were blunt-ended and ligated with EcoRI linkers as in Example 1. The resultant fragments were analyzed by electrophoresis (5-10V/cm) on 1.2% agarose gel, using PhiX174/HaeIII and lambda/HindIII size markers. The 100-300 bp fraction was eluted onto NA45 strips (Schleicher and Schuell), which were then placed into 1.5 ml microtubes with eluting solution (1 M NaCl, 50 mM arginine, pH 9.0), and incubated at 67°C for 30-60 minutes. The eluted DNA was phenol/chloroform extracted and then precipitated with two volumes of ethanol. The pellet was resuspended in 20 µl TE (0.01 M Tris HC1, pH 7.5, 0.001 M EDTA).

### B. Cloning in an Expression Vector

Lambda gt11 phage vector (Huynh) was obtained from Promega Biotec (Madison, WI). This cloning vector has a unique EcoRI cloning site 53 base pairs upstream from the beta-galactosidase translation termination codon. The genomic fragments from above, provided either directly from coding sequences (Example 5) or after amplification of cDNA (Example 4), were introduced into the EcoRI site by mixing 0.5-1.0 µg EcoRI-cleaved gt11, 0.3-3 µl of the above sized fragments, 0.5 µl 10X ligation buffer (above), 0.5 µl ligase (200 units), and distilled water to 5 µl. The mixture was incubated overnight at 14°C, followed by in vitro packaging, according to standard methods (Maniatis, pp. 256-268).

The packaged phage were used to infect E. coli strain KM392, obtained from Dr. Kevin Moore, DNAX (Palo Alto, CA). Alternatively, E. Coli strain Y1090, available from the American Type Culture Collection (ATCC #37197), could be used. The infected bacteria were plated and the resultant colonies were checked for loss of beta-galactosidase activity-(clear plaques) in the presence of X-gal using a standard X-gal substrate plaque assay method (Maniatis). About 50% of the phage plaques showed loss of beta-galactosidase enzyme activity (recombinants).

### C. Screening for ET-NANB Recombinant Proteins

ET-NANB convalescent antiserum was obtained from patients infected during documented ET-NANB outbreaks in Mexico, Borneo, Pakistan, Somalia, and Burma. The sera were immunoreactive with VLPs in stool specimens from each of several other patients with ET-NANB hepatitis.

A lawn of E. coli KM392 cells infected with about 104 pfu of the phage stock from above was prepared on a 150 mm plate and incubated, inverted, for 5-8 hours at 37°C. The lawn was overlaid with a nitrocellulose sheet, causing transfer of expressed ETNANB recombinant protein from the plaques to the paper. The plate and filter were indexed for matching corresponding plate and filter positions.

The filter was washed twice in TBST buffer (10 mM Tris, pH 8.0, 150 mM NaCl, 0.05% Tween 20), blocked with AIB (TBST buffer with 1% gelatin), washed again in TBST, and incubated overnight after addition of antiserum (diluted to 1:50 in AIB, 12-15 ml/plate). The sheet was washed twice in TBST and then contacted with enzyme-labeled anti-human antibody to attach the labeled antibody at filter sites containing antigen recognized by the antiserum. After a final washing, the filter was developed in a substrate medium containing 33 µl NBT (50 mg/ml stock solution maintained at 4°C) mixed with 16 µl BCIP (50 mg/ml stock solution maintained at 4°C) in 5 ml of alkaline phosphatase buffer (100 mM Tris, 9.5, 100 mM NaCl, 5 mM MgC12). Purple color appeared at points of antigen production, as recognized by the antiserum.

### D. Screening Plating

The areas of antigen production determined in the previous step were replated at about 100-200 pfu on an 82 mm plate. The above steps, beginning with a 5-8 hour incubation, through NBT-BCIP development, were repeated in order to plaque purify phage secreting an antigen capable of reacting with the ET-NANB antibody. The identified plaques were picked and eluted in phage buffer (Maniatis, p. 443).

### E. Epitope Identification

A series of subclones derived from the original pTZKF1 (ETI.1) plasmid from Example 2 were isolated using the same techniques described above. Each of these five subclones were immunoreactive with a pool of anti-ET antisera noted in C. The subclones contained short sequences from the "reverse" sequence set forth previously. The beginning and ending points of the sequences in the subclones (relative to the full "reverse" sequence), are identified in the table below.

| TABLE 1 | | |
|---|---|---|
| Subclone | Position in | "Reverse" Sequence |
| | 5'-end | 3'-end |
| Y1 | 522 | 643 |
| Y2 | 594 | 667 |
| Y3 | 508 | 665 |
| Y4 | 558 | 752 |
| Y5 | 545 | 665 |

Since all of the gene sequences identified in the table must contain the coding sequence for the epitope, it is apparent that the coding sequence for the epitope falls in the region between nucleotide 594 (5'-end) and 643 (3'-end). Genetic sequences equivalent to and complementary to this relatively short sequence are therefore particularly preferred aspects of the present invention, as are peptides produced using this coding region.

A second series of clones identifying an altogether different epitope was isolated with only Mexican serum.

| TABLE 2 | | |
|---|---|---|
| Subclone | Position in | "Forward" Sequence |
| | 5'end | 3' end |
| ET 2-2 | 2 | 193 |
| ET 8-3 | 2 | 135 |
| ET 9-1 | 2 | 109 |
| ET 13-1 | 2 | 101 |

The coding system for this epitope falls between nucleotide 2 (S -end) and 101 (3 -end). Genetic sequences related to this short sequence are therefore also preferred, as are peptides produced using this coding region.

Two particularly preferred subclones for use in preparing polypeptides containing epitopes specific for HEV are the 406.3-2 and 406.4-2 clones whose sequences are set forth above. These sequences were isolated from an amplified cDNA library derived from a Mexican stool. Using the techniques described in this section, polypeptides expressed by these clones have been tested for immunoreactivity against a number of different human HEV-positive sera obtained from sources around the world. As shown in Table 3 below, 8 sera immunoreactive with the polypeptide expressed by the 406.4-2, and 6 sera immunoreacted with polypeptide expressed by the 406.3-2 clone.

For comparison, the Table also shows reactivity of the various human sera with the Y2 clone identified in Table 1 above. Only one of the sera reacted with the polypeptide expressed by this clone. No immunoreactivity was seen for normal expression products of the gt11 vector.

| Table 3 | | | | | | |
|---|---|---|---|---|---|---|
| Immunoreactivity of HEV Recombinant Proteins: Human Sera | | | | | | |
| Sera | Source | Stage¹ | 406.3-2 | 406.4-2 | Y2 | λgt11 |
| FVH-21 | Burma | A | - | - | - | - |
| FVH-8 | Burma | A | - | + | + | - |
| SOM-19 | Somalia | A | + | + | - | - |
| SOM-20 | Somalia | A | + | + | - | - |
| IM-35 | Borneo | A | + | + | - | - |
| IM-36 | Borneo | A | - | - | - | - |
| PAK-1 | Pakistan | A | + | + | - | - |
| FFI-4 | Mexico | A | + | + | - | - |
| FFI-125 | Mexico | A | - | + | - | - |
| F 387 IC | Mexico | C | + | + | ND | - |
| Normal | U.S.A. | - | - | - | - | - |
| | | | | | | |

| | | | | | | |
|---|---|---|---|---|---|---|
| ¹A = acute; C = convalescent | | | | | | |

While the invention has been described with reference to particular embodiments, methods, construction and use, it will be apparent to those skilled in the art that various changes and modifications can be made without departing from the invention.

## Claims

1. A protein which is
(a) immunoreactive with antibodies present in individuals infected with enterically transmitted nonA/nonB hepatitis; and
(b) either encoded by
(i) the genetic sequence 406.3-2 identified by SEQ ID No. 15;
(ii) a genetic sequence having at most 25-30% base pair mismatches with (i); or
(iii) a fragment of (i), or (ii) comprising at least 12 consecutive nucleotides.

2. An in vitro method of detecting infection by enterically transmitted nonA/nonB hepatitis viral agent in a test individual, comprising:
providing a peptide antigen which is (a) immunoreactive with antibodies from individuals infected with enterically transmitted nonA/nonB hepatitis and (b) either encoded by
(i) the genetic sequence 406.3-2 identified by SEQ ID No. 15;
(ii) a genetic sequence having at most 25-30% base pair mismatches with (i); or
(iii) a fragment of (i) or (ii) comprising at least 12 consecutive nucleotides; reacting serum from the test individual with such antigen; and
examining the antigen for the presence of bound antibody.

3. A method according to claim 2 wherein the serum antibody is an IgM or IgG antibody, or a mixture of both, the antigen provided is attached to a support, said reacting includes contacting said serum with the support and said examining includes reacting the support and bound serum antibody with a reporter-labelled anti-human antibody.

4. A kit for ascertaining the presence of serum antibodies which are diagnostic of enterically transmitted nonA/nonB hepatitis infection comprising:
a support with surface bound peptide antigen providing a peptide antigen which is (a) immunoreactive with antibodies present in individuals infected with enterically transmitted nonA/nonB hepatitis and (b) either encoded by
(i) the genetic sequence 406.3-2 identified by SEQ ID No. 15;
(ii) a genetic sequence having at most 25-30% base pair mismatches with (i) or a fragment of (i) comprising at least 12 consecutive nucleotides.

5. A DNA molecule which comprises the genetic sequence 406.3-2 as shown in SEQ ID No. 15 or a fragment thereof, wherein said fragment comprises at least 12 consecutive nucleotides.

6. A vector which comprises a DNA molecule according to claim 5.

7. A host cell which comprises a vector according to claim 6.

8. A kit comprising, in a container or separate containers, a pair of single-strand primers derived from non-homologous regions of opposite strands of a DNA duplex fragment derived from an enterically transmitted viral hepatitis agent whose genome contains a region which is homologous to the 1.33 kb DNA EcoRI insert present in plasmid pTZKF1 (ET1.1) carried in E. coli strain BB4 and having ATCC deposit no. 67717, said primers comprising a fragment of the genetic sequence 406.3-2 as shown in SEQ ID No. 15, and said fragment comprising at least 12 consecutive nucleotides.

9. A method for detecting the presence of enterically transmitted nonA/nonB hepatitis viral agent in a test individual, comprising:
preparing a mixture of duplex DNA fragments derived from a suitable sample derived from the individual;
denaturing the duplex fragments, adding to the denatured DNA fragments a pair of single stranded primers derived from non-homologous regions of opposite strands of a DNA duplex fragment derived from an enterically transmitted viral hepatitis agent whose genome contains a region which is homologous to the 1.33 kb DNA EcoRI insert present in plasmid pTZKF1 (ET1.1) carried in E. coli strain BB4 and having ATCC deposit no. 67717, said primers comprising a fragment of the genetic sequence 406.3-2 as shown in SEQ ID No. 15, and said fragment comprising at least 12 consecutive nucleotides,
hybridising said primers to homologous-sequence region of opposite strands of said duplex DNA fragments;
reaching the primed fragment strands with DNA polymerase in the presence of DNA nucleotides, to turn new DNA duplexes containing the primer sequences, and repeating said denaturing, adding, hybridising and reacting steps, until a desired degree of amplification of sequences is achieved.

10. A method according to claim 9 for detecting the presence of a viral agent in a sample of cultured cells infected with the agent.

11. A vaccine for immunising an individual against HEV comprising, in a pharmalogically acceptable adjuvant a protein encoded by a genetic sequence of 406.3-2.

## Patentansprüche

1. Protein, das
(a) mit Antikörpern, die in Individuen vorhanden sind, die mit enterisch übertragener Non-A-/Non-B-Hepatitis infiziert sind, immunreaktiv ist; sowie
(b) entweder von
(i) der genetischen Sequenz 406.3-2, identifiziert durch Seq.-ID Nr. 15;
(ii) von einer genetischen Sequenz mit höchstens 25-30 % Basenpaar-Fehlpaarungen mit (i); oder
(iii) von einem Fragment aus (i) oder (ii), umfassend zumindest 12 aufeinander folgende Nucleotide,
kodiert wird.

2. In-vitro-Verfahren zur Detektion einer Infektion durch ein enterisch übertragenes virales Non-A-/Non-B-Hepatitis-Agens in einem Test-Individuum, umfassend:
die Bereitstellung eines Peptid-Antigens, das (a) mit Antikörpern immunreaktiv ist, die von Individuen stammen, die mit enterisch übertragener Non-A-/Non-B-Hepatitis infiziert sind, und (b) entweder von
(i) der genetischen Sequenz 406.3-2, identifiziert durch Seq.-ID Nr. 15;
(ii) von einer genetischen Sequenz mit höchstens 25-30 % Basenpaar-Fehlpaarungen mit (i); oder
(iii) von einem Fragment aus (i) oder (ii), umfassend zumindest 12 aufeinander folgende Nucleotide;
kodiert wird;
das Umsetzen von Serum des Test-Individuums mit diesem Antigen; und
das Untersuchen des Antigens auf die Gegenwart von gebundenem Antikörper.

3. Verfahren nach Anspruch 2, worin der Serum-Antikörper ein IgM- oder IgG-Antikörper oder ein Gemisch aus beiden ist, das bereitgestellte Antigen an einen Träger gebunden ist, wobei das Umsetzen das Kontaktieren des Serums mit dem Träger umfasst und das Untersuchen das Umsetzen des Trägers und des gebundenen Serum-Antikörpers mit einem reportermarkierten Anti-Mensch-Antikörper umfasst.

4. Set zur Feststellung der Gegenwart von Serum-Antikörpern, die für eine enterisch übertragene Non-A-/Non-B-Hepatitis-Infektion diagnostisch sind, umfassend:
einen Träger mit oberflächengebundenem Peptid-Antigen, das ein Peptid-Antigen bereitstellt, das (a) mit Antikörpern immunreaktiv ist, die in Individuen vorhanden sind, die mit enterisch übertragener Non-A-/Non-B-Hepatitis infiziert sind, und (b) entweder von
(i) der genetischen Sequenz 406.3-2, identifiziert durch Seq.-ID Nr. 15;
(ii) von einer genetischen Sequenz mit höchstens 25-30 % Basenpaar-Fehlpaarungen mit (i) oder einem Fragment aus (i), umfassend zumindest 12 aufeinander folgende Nucleotide;
kodiert wird.

5. DNA-Molekül, das die genetische Sequenz 406.3-2, dargestellt in Seq.-ID Nr. 15, oder ein Fragment davon umfasst, worin das Fragment zumindest 12 aufeinander folgende Nucleotide umfasst.

6. Vektor, der ein DNA-Molekül nach Anspruch 5 umfasst.

7. Wirtszelle, die einen Vektor nach Anspruch 6 umfasst.

8. Set, umfassend in einem Behälter oder in separaten Behältern ein Paar Einzelstrang-Primer, die von nicht-homologen Regionen entgegengesetzter Stränge eines DNA-Duplex-Fragments abstammen, das von einem enterisch übertragenen viralen Hepatitis-Agens abstammt, dessen Genom eine Region enthält, die zum 1,33-kb-DNA-EcoRl-Insert homolog ist, das im Plasmid pTZKF1 (ET1.1), vorhanden im E.-coli-Stamm BB4, vorliegt und die ATCC-Hinterlegungsnr. 67717 aufweist, wobei die Primer ein Fragment der genetischen Sequenz 406.3-2, wie in Seq.-ID Nr. 15 dargestellt, umfassen und das Fragment zumindest 12 aufeinander folgende Nucleotide umfasst.

9. Verfahren zur Detektion der Gegenwart eines enterisch übertragenen viralen Non-A-/Non-B-Hepatitis-Agens in einem Test-Individuum, umfassend:
das Herstellen eines Gemisches aus Duplex-DNA-Fragmenten, die von einer geeigneten Probe stammen, die vom Individuum stammt;
das Denaturieren der Duplex-Fragmente, das Hinzufügen eines Paars einzelsträngiger Primer, die aus den nicht-homologen Regionen entgegengesetzter Stränge eines DNA-Duplex-Fragments stammen, das von einem enterisch übertragenen viralen Hepatitis-Agens abstammt, dessen Genom eine Region enthält, die zum 1,33-kb-DNA-EcoRl-Insert homolog ist, das im Plasmid pTZKF1 (ET1.1), vorhanden im E.-coli-Stamm BB4, vorliegt und die ATCC-Hinterlegungsnr. 67717 aufweist, zu den denaturierten DNA-Fragmenten, wobei die Primer ein Fragment der genetischen Sequenz 406.3-2, wie in Seq.-ID Nr. 15 dargestellt, umfassen und das Fragment zumindest 12 aufeinander folgende Nucleotide umfasst,
das Hybridisieren der Primer an eine homologe Sequenzregion entgegengesetzter Stränge der Duplex-DNA-Fragmente;
das Umsetzen der geprimten Fragment-Stränge mit DNA-Polymerase in Gegenwart von DNA-Nucleotiden, um neue DNA-Duplexe zu wickeln, welche die Primer-Sequenzen enthalten, und das Wiederholen der Schritte des Denaturierens, des Hinzufügens, des Hybridisierens und des Umsetzens, bis ein gewünschtes Ausmaß an Sequenzamplifikation erreicht ist.

10. Verfahren nach Anspruch 9 zur Detektion der Gegenwart eines viralen Agens in einer Probe gezüchteter Zellen, die mit dem Agens infiziert sind.

11. Vakzine zur Immunisierung eines Individuums gegen HEV, umfassend, in einem pharmakologisch annehmbaren Adjuvans, ein Protein, das von einer genetischen Sequenz 406.3-2 kodiert wird.

## Revendications

1. Protéine qui est
(a) immunoréactive avec les anticorps présents chez des individus infectés d'une hépatite nonA/nonB transmise par voie entérique ; et
(b) soit codée par
(i) la séquence génétique 406.3-2 identifiée par SEQ ID NO. 15 ;
(ii) une séquence génétique ayant au plus 25-30% de défauts d'appariement de paires de bases avec (i) ; ou
(iii) un fragment de (i) ou (ii) comprenant au moins 12 nucléotides consécutifs.

2. Méthode in vitro pour la détection d'une infection par un agent viral de l'hépatite nonA/nonB transmis par voie entérique chez un individu testé, consistant à
prévoir un antigène peptidique qui est (a) immunoréactif avec les anticorps d'individus infectés de l'hépatite nonA/nonB transmise par voie entérique et (b) soit codée par
(i) la séquence génétique 406.3-2 identifiée par SEQ ID NO. 15 ;
(ii) une séquence génétique ayant au plus 25-30% de défauts d'appariement de paires de bases avec (i) ; ou
(iii) un fragment de (i) ou (ii) comprenant au moins 12 nucléotides consécutifs,
en faisant réagir le sérum de l'individu testé avec cet antigène ; et
en examinant l'antigène à la recherche de la présence de l'anticorps lié.

3. Méthode selon la revendication 2, où l'anticorps du sérum est un anticorps de IgM ou IgG ou un mélange des deux, l'antigène prévu est attaché à un support, ladite réaction comprend la mise en contact dudit sérum avec le support et ledit examen comprend la réaction du support et de l'anticorps du sérum lié avec un anticorps anti-humain marqué par un reporteur.

4. Kit pour déterminer la présence d'anticorps dans le sérum qui sont le diagnostic d'une infection par une hépatite nonA/nonB transmise par voie entérique comprenant :
un support avec un antigène peptidique lié en surface produisant un antigène peptidique qui est (a) immunoréactif avec des anticorps présents chez des individus infectés d'une hépatite nonA/nonB transmise par voie entérique et (b) soit codée par
(i) la séquence génétique 406.3-2 identifiée par SEQ ID NO. 15 ;
(ii) une séquence génétique ayant au plus 25-30% de défauts d'appariement de paires de bases avec (i) ; ou
(iii) un fragment de (i) ou (ii) comprenant au moins 12 nucléotides consécutifs.

5. Molécule d'ADN qui comprend la séquence génétique 406.3-2 telle que montrée à SEQ ID NO. 15 ou un fragment de celle-ci où ledit fragment comprend au moins 12 nucléotides consécutifs.

6. Vecteur qui comprend une molécule d'ADN selon la revendication 5.

7. Cellule hôte qui comprend un vecteur selon la revendication 6.

8. Kit comprenant, dans un conteneur ou des conteneurs séparés à une paire d'amorces monocaténaires dérivées de régions non homologues de brins opposés d'un fragment duplex d'ADN dérivé d'un agent d'hépatite virale transmis par voie entérique dont le génome contient une région qui est homologue à l'insert EcoRI d'ADN de 1,33 kb présent dans le plasmide pTZKF1 (ET1.1) porté dans la souche BB4 de E. coli et ayant un dépôt ATCC N° 67717, lesdites amorces comprenant un fragment de la séquence génétique 406.3-2 telle que montrée à SEQ ID NO. 15, et ledit fragment comprenant au moins 12 nucléotides consécutifs.

9. Méthode pour la détection de la présence d'un agent viral d'hépatite nonA/nonB transmis par voie entérique chez un individu testé comprenant :
la préparation d'un mélange de fragments d'ADN en duplex dérivés d'un échantillon approprié dérivé de l'individu ;
la dénaturation des fragments en duplex, l'addition aux fragments dénaturés d'ADN d'une paire d'amorces monocaténaires dérivées des régions non homologues de brins opposés d'un fragment en duplex d'ADN dérivé d'un agent d'hépatite virale transmis par voie entérique dont le génome contient une région qui est homologue à l'insert EcoRI d'ADN de 1,33 kb présent dans le plasmide pTZKF1 (ET1.1) porté dans la souche BB4 de E. Coli et ayant le dépôt ATCC N° 67717, lesdites amorces comprenant un fragment de la séquence génétique 406.3-02 comme montré à SEQ ID NO. 15 et lesdits fragments comprenant au moins 12 nucléotides consécutifs,
l'hybridation desdites amorces à une région de séquence homogue des brins opposés desdits fragments d'ADN en duplex,
la réaction des brins de fragments amorcés avec l'ADN polymérase en présence d'ADN nucléotides pour tourner de nouveaux duplex d'ADN contenant les séquences d'amorce et la répétition desdites étapes de dénaturation, addition, hybridation et réaction jusqu'à ce qu'un degré souhaité d'amplification des séquences soit atteint.

10. Méthode selon la revendication 9 pour la détection de la présence d'un agent viral dans un échantillon de cellules cultivées infectées de l'agent.

11. Vaccin pour immuniser un individu contre HEV comprenant un adjuvant pharmaceutiquement acceptable une protéine codée par une séquence génétique de 406.3-2.


### SEQUENCE LISTING

<110> Genelabs Technologies, Inc.
<110> Government of The United States of America, as represented by The Secretary of The Department of Health and Human Services and His Successors
<120> Enterically Transmitted Non-A/Non-B Hepatitis Viral Agent and Characteristic Epitopes Thereof
<130> SMK/FP5066790
<140> EP 91908163.8
   <141> 1991-04-05
<150> PCT/US91/02368
   <151> 1991-04-05
<150> US 07/505,888
   <151> 1990-04-05
<160> 55
<170> PatentIn Ver. 3.3
<210> 1
   <211> 1295
   <212> DNA
   <213> Hepatitis E virus
<400> 1
   agacctgtcc ctgttgcagc tgttctacca ccctgccccg agctcgaaca gggccttctc 60
   tacctgcccc aggagctcac cacctgtgat agtgtcgtaa catttgaatt aacagacatt 120
   gtgcactgcc gcatggccgc cccgagccag cgcaaggccg tgctgtccac actcgtgggc 180
   cgctacggcg gtcgcacaaa gctctacaat gcttcccact ctgatgttcg cgactctctc 240
   gcccgtttta tcccggccat tggccccgta caggttacaa cttgtgaatt gtacgagcta 300
   gtggaggcca tggtcgagaa gggccaggat ggctccgccg tccttgagct tgatctttgc 360
   aaccgtgacg tgtccaggat caccttcttc cagaaagatt gtaacaagtt caccacaggt 420
   gagaccattg cccatggtaa agtgggccag ggcatctcgg cctggagcaa gaccttctgc 480
   gccctctttg gcccttggtt ccgcgctatt gagaaggcta ttctggccct gctccctcag 540
   ggtgtgtttt acggtgatgc ctttgatgac accgtcttct cggcggctgt ggccgcagca 600
   aaggcatcca tggtgtttga gaatgacttt tctgagtttg actccaccca gaataacttt 660
   tctctgggtc tagagtgtgc tattatggag gagtgtggga tgccgcagtg gctcatccgc 720
   ctgtatcacc ttataaggtc tgcgtggatc ttgcaggccc cgaaggagtc tctgcgaggg 780
   ttttggaaga aacactccgg tgagcccggc actcttctat ggaatactgt ctggaatatg 840
   gccgttatta cccactgtta tgacttccgc gattttcagg tggctgcctt taaaggtgat 900
   gattcgatag tgctttgcag tgagtatcgt cagagtccag gagctgctgt cctgatcgcc 960
   ggctgtggct tgaagttgaa ggtagatttc cgcccgatcg gtttgtatgc aggtgttgtg 1020
   gtggcccccg gccttggcgc gctccctgat gttgtgcgct tcgccggccg gcttaccgag 1080
   aagaattggg gccctggccc tgagcgggcg gagcagctcc gcctcgctgt tagtgatttc 1140
   ctccgcaagc tcacgaatgt agctcagatg tgtgtggatg ttgtttcccg tgtttatggg 1200
   gtttcccctg gactcgttca taacctgatt ggcatgctac aggctgttgc tgatggcaag 1260
   gcacatttca ctgagtcagt aaaaccagtg ctcga                            1295
<210> 2
   <211> 431
   <212> PRT
   <213> Hepatitis E virus
<400> 2
   Arg Pro Val Pro Val Ala Ala Val Leu Pro Pro Cys Pro Glu Leu Glu
    1               5                  10                  15
   Gln Gly Leu Leu Tyr Leu Pro Gln Glu Leu Thr Thr Cys Asp Ser Val
                20                  25                  30
   Val Thr Phe Glu Leu Thr Asp Ile Val His Cys Arg Met Ala Ala Pro
            35                  40                  45
   Ser Gln Arg Lys Ala Val Leu Ser Thr Leu Val Gly Arg Tyr Gly Gly
        50                  55                  60
   Arg Thr Lys Leu Tyr Asn Ala Ser His Ser Asp Val Arg Asp Ser Leu
    65                  70                  75                  80
   Ala Arg Phe Ile Pro Ala Ile Gly Pro Val Gln Val Thr Thr Cys Glu
                    85                  90                  95
   Leu Tyr Glu Leu Val Glu Ala Met Val Glu Lys Gly Gln Asp Gly Ser
               100                 105                 110
   Ala Val Leu Glu Leu Asp Leu Cys Asn Arg Asp Val Ser Arg Ile Thr
           115                 120                 125
   Phe Phe Gln Lys Asp Cys Asn Lys Phe Thr Thr Gly Glu Thr Ile Ala
       130                 135                 140
   His Gly Lys Val Gly Gln Gly Ile Ser Ala Trp Ser Lys Thr Phe Cys
   145                 150                 155                 160
   Ala Leu Phe Gly Pro Trp Phe Arg Ala Ile Glu Lys Ala Ile Leu Ala
                   165                 170                 175
   Leu Leu Pro Gln Gly Val Phe Tyr Gly Asp Ala Phe Asp Asp Thr Val
               180                 185                 190
   Phe Ser Ala Ala Val Ala Ala Ala Lys Ala Ser Met Val Phe Glu Asn
           195                 200                 205
   Asp Phe Ser Glu Phe Asp Ser Thr Gln Asn Asn Phe Ser Leu Gly Leu
       210                 215                 220
   Glu Cys Ala Ile Met Glu Glu Cys Gly Met Pro Gln Trp Leu Ile Arg
   225                 230                 235                 240
   Leu Tyr His Leu Ile Arg Ser Ala Trp Ile Leu Gln Ala Pro Lys Glu
                   245                 250                 255
   Ser Leu Arg Gly Phe Trp Lys Lys His Ser Gly Glu Pro Gly Thr Leu
               260                 265                 270
   Leu Trp Asn Thr Val Trp Asn Met Ala Val Ile Thr His Cys Tyr Asp
           275                 280                 285
   Phe Arg Asp Phe Gln Val Ala Ala Phe Lys Gly Asp Asp Ser Ile Val
       290                 295                 300
   Leu Cys Ser Glu Tyr Arg Gln Ser Pro Gly Ala Ala Val Leu Ile Ala
   305                 310                 315                 320
   Gly Cys Gly Leu Lys Leu Lys Val Asp Phe Arg Pro Ile Gly Leu Tyr
   325 330 335
   Ala Gly Val Val Val Ala Pro Gly Leu Gly Ala Leu Pro Asp Val Val
   340 345 350
   Arg Phe Ala Gly Arg Leu Thr Glu Lys Asn Trp Gly Pro Gly Pro Glu
   355 360 365
   Arg Ala Glu Gln Leu Arg Leu Ala Val Ser Asp Phe Leu Arg Lys Leu
   370 375 380
   Thr Asn Val Ala Gln Met Cys Val Asp Val Val Ser Arg Val Tyr Gly
   385 390 395 400
   Val Ser Pro Gly Leu Val His Asn Leu Ile Gly Met Leu Gln Ala Val
   405 410 415
   Ala Asp Gly Lys Ala His Phe Thr Glu Ser Val Lys Pro Val Leu
   420 425 430
<210> 3
   <211> 32
   <212> PRT
   <213> Hepatitis E virus
<400> 3
   Asp Leu Ser Leu Leu Gln Leu Phe Tyr His Pro Ala Pro Ser Ser Asn
   1 5 10 15
   Arg Ala Phe Ser Thr Cys Pro Arg Ser Ser Pro Pro Val Ile Val Ser
   20 25 30
<210> 4
   <211> 28
   <212> PRT
   <213> Hepatitis E virus
<400> 4
   Thr Cys Pro Cys Cys Ser Cys Ser Thr Thr Leu Pro Arg Ala Arg Thr
   1 5 10 15
   Gly Pro Ser Leu Pro Ala Pro Gly Ala His His Leu
   20 25
<210> 5
   <211> 1295
   <212> DNA
   <213> Hepatitis E virus
<400> 5
   tcgagcactg gttttactga ctcagtgaaa tgtgccttgc catcagcaac agcctgtagc 60
   atgccaatca ggttatgaac gagtccaggg gaaaccccat aaacacggga aacaacatcc 120
   acacacatct gagctacatt cgtgagcttg cggaggaaat cactaacagc gaggcggagc 180
   tgctccgccc gctcagggcc agggccccaa ttcttctcgg taagccggcc ggcgaagcgc 240
   acaacatcag ggagcgcgcc aaggccgggg gccaccacaa cacctgcata caaaccgatc 300
   gggcggaaat ctaccttcaa cttcaagcca cagccggcga tcaggacagc agctcctgga 360
   ctctgacgat actcactgca aagcactatc gaatcatcac ctttaaaggc agccacctga 420
   aaatcgcgga agtcataaca gtgggtaata acggccatat tccagacagt attccataga 480
   agagtgccgg gctcaccgga gtgtttcttc caaaaccctc gcagagactc cttcggggcc 540
   tgcaagatcc acgcagacct tataaggtga tacaggcgga tgagccactg cggcatccca 600
   cactcctcca taatagcaca ctctagaccc agagaaaagt tattctgggt ggagtcaaac 660
   tcagaaaagt cattctcaaa caccatggat gcctttgctg cggccacagc cgccgagaag 720
   acggtgtcat caaaggcatc accgtaaaac acaccctgag ggagcagggc cagaatagcc 780
   ttctcaatag cgcggaacca agggccaaag agggcgcaga aggtcttgct ccaggccgag 840
   atgccctggc ccactttacc atgggcaatg gtctcacctg tggtgaactt gttacaatct 900
   ttctggaaga aggtgatcct ggacacgtca cggttgcaaa gatcaagctc aaggacggcg 960
   gagccatcct ggcccttctc gaccatggcc tccactagct cgtacaattc acaagttgta 1020
   acctgtacgg ggccaatggc cgggataaaa cgggcgagag agtcgcgaac atcagagtgg 1080
   gaagcattgt agagctttgt gcgaccgccg tagcggccca cgagtgtgga cagcacggcc 1140
   ttgcgctggc tcggggcggc catgcggcag tgcacaatgt ctgttaattc aaatgttacg 1200
   acactatcac aggtggtgag ctcctggggc aggtagagaa ggccctgttc gagctcgggg 1260
   cagggtggta gaacagctgc aacagggaca ggtct                            1295
<210> 6
   <211> 7195
   <212> DNA
   <213> Hepatitis E virus
<220>
   <221> CDS
   <222> (28)..(5106)
<400> 6
   aggcagacca catatgtggt cgatgcc atg gag gcc cat cag ttt att aag gct 54
                                 Met Glu Ala His Gln Phe Ile Lys Ala
                                  1               5
   cct ggc atc act act gct att gag cag gct gct cta gca gcg gcc aac    102
   Pro Gly Ile Thr Thr Ala Ile Glu Gln Ala Ala Leu Ala Ala Ala Asn
    10                  15                  20                  25
   tct gcc ctg gcg aat gct gtg gta gtt agg cct ttt ctc tct cac cag    150
   Ser Ala Leu Ala Asn Ala Val Val Val Arg Pro Phe Leu Ser His Gln
                   30                  35                  40
   cag att gag atc ctc att aac cta atg caa cct cgc cag ctt gtt ttc    198
   Gln Ile Glu Ile Leu Ile Asn Leu Met Gln Pro Arg Gln Leu Val Phe
               45                  50                  55
   cgc ccc gag gtt ttc tgg aat cat ccc atc cag cgt gtc atc cat aac    246
   Arg Pro Glu Val Phe Trp Asn His Pro Ile Gln Arg Val Ile His Asn
           60                  65                  70
   gag ctg gag ctt tac tgc cgc gcc cgc tcc ggc cgc tgt ctt gaa att    294
   Glu Leu Glu Leu Tyr Cys Arg Ala Arg Ser Gly Arg Cys Leu Glu Ile
       75                  80                  85
   ggc gcc cat ccc cgc tca ata aat gat aat cct aat gtg gtc cac cgc    342
   Gly Ala His Pro Arg Ser Ile Asn Asp Asn Pro Asn Val Val His Arg
    90                  95                 100                 105
   tgc ttc ctc cgc cct gtt ggg cgt gat gtt cag cgc tgg tat act gct    390
   Cys Phe Leu Arg Pro Val Gly Arg Asp Val Gln Arg Trp Tyr Thr Ala
                   110                 115                 120
   ccc act cgc ggg ccg gct gct aat tgc cgg cgt tcc gcg ctg cgc ggg    438
   Pro Thr Arg Gly Pro Ala Ala Asn Cys Arg Arg Ser Ala Leu Arg Gly
               125                 130                 135
   ctt ccc gct gct gac cgc act tac tgc ctc gac ggg ttt tct ggc tgt    486
   Leu Pro Ala Ala Asp Arg Thr Tyr Cys Leu Asp Gly Phe Ser Gly Cys
           140                 145                 150
   aac ttt ccc gcc gag act ggc atc gcc ctc tac tcc ctt cat gat atg    534
   Asn Phe Pro Ala Glu Thr Gly Ile Ala Leu Tyr Ser Leu His Asp Met
       155                 160                 165
   tca cca tct gat gtc gcc gag gcc atg ttc cgc cat ggt atg acg cgg    582
   Ser Pro Ser Asp Val Ala Glu Ala Met Phe Arg His Gly Met Thr Arg
   170                 175                 180                 185
   ctc tat gcc gcc ctc cat ctt ccg cct gag gtc ctg ctg ccc cct ggc    630
   Leu Tyr Ala Ala Leu His Leu Pro Pro Glu Val Leu Leu Pro Pro Gly
                   190                 195                 200
   aca tat cgc acc gca tcg tat ttg cta att cat gac ggt agg cgc gtt    678
   Thr Tyr Arg Thr Ala Ser Tyr Leu Leu Ile His Asp Gly Arg Arg Val
               205                 210                 215
   gtg gtg acg tat gag ggt gat act agt gct ggt tac aac cac gat gtc    726
   Val Val Thr Tyr Glu Gly Asp Thr Ser Ala Gly Tyr Asn His Asp Val
           220                 225                 230
   tcc aac ttg cgc tcc tgg att aga acc acc aag gtt acc gga gac cat    774
   Ser Asn Leu Arg Ser Trp Ile Arg Thr Thr Lys Val Thr Gly Asp His
       235                 240                 245
   ccc ctc gtt atc gag cgg gtt agg gcc att ggc tgc cac ttt gtt ctc    822
   Pro Leu Val Ile Glu Arg Val Arg Ala Ile Gly Cys His Phe Val Leu
   250                 255                 260                 265
   ttg ctc acg gca gcc ccg gag cca tca cct atg cct tat gtt cct tac    870
   Leu Leu Thr Ala Ala Pro Glu Pro Ser Pro Met Pro Tyr Val Pro Tyr
                   270                 275                 280
   ccc cgg tct acc gag gtc tat gtc cga tcg atc ttc ggc ccg ggt ggc    918
   Pro Arg Ser Thr Glu Val Tyr Val Arg Ser Ile Phe Gly Pro Gly Gly
               285                 290                 295
   acc cct tcc tta ttc cca acc tca tgc tcc act aag tcg acc ttc cat    966
   Thr Pro Ser Leu Phe Pro Thr Ser Cys Ser Thr Lys Ser Thr Phe His
           300                 305                 310
   gct gtc cct gcc cat att tgg gac cgt ctt atg ctg ttc ggg gcc acc    1014
   Ala Val Pro Ala His Ile Trp Asp Arg Leu Met Leu Phe Gly Ala Thr
       315                 320                 325
   ttg gat gac caa gcc ttt tgc tgc tcc cgt tta atg acc tac ctt cgc    1062
   Leu Asp Asp Gln Ala Phe Cys Cys Ser Arg Leu Met Thr Tyr Leu Arg
   330                 335                 340                 345
   ggc att agc tac aag gtc act gtt ggt acc ctt gtg gct aat gaa ggc    1110
   Gly Ile Ser Tyr Lys Val Thr Val Gly Thr Leu Val Ala Asn Glu Gly
                   350                 355                 360
   tgg aat gcc tct gag gac gcc ctc aca gct gtt atc act gcc gcc tac    1158
   Trp Asn Ala Ser Glu Asp Ala Leu Thr Ala Val Ile Thr Ala Ala Tyr
               365                 370                 375
   ctt acc att tgc cac cag cgg tat ctc cgc acc cag gct ata tcc aag    1206
   Leu Thr Ile Cys His Gln Arg Tyr Leu Arg Thr Gln Ala Ile Ser Lys
           380                 385                 390
   ggg atg cgt cgt ctg gaa cgg gag cat gcc cag aag ttt ata aca cgc    1254
   Gly Met Arg Arg Leu Glu Arg Glu His Ala Gln Lys Phe Ile Thr Arg
       395                 400                 405
   ctc tac agc tgg ctc ttc gag aag tcc ggc cgt gat tac atc cct ggc    1302
   Leu Tyr Ser Trp Leu Phe Glu Lys Ser Gly Arg Asp Tyr Ile Pro Gly
   410                 415                 420                 425
   cgt cag ttg gag ttc tac gcc cag tgc agg cgc tgg ctc tcc gcc ggc    1350
   Arg Gln Leu Glu Phe Tyr Ala Gln Cys Arg Arg Trp Leu Ser Ala Gly
                   430                 435                 440
   ttt cat ctt gat cca cgg gtg ttg gtt ttt gac gag tcg gcc ccc tgc    1398
   Phe His Leu Asp Pro Arg Val Leu Val Phe Asp Glu Ser Ala Pro Cys
               445                 450                 455
   cat tgt agg acc gcg atc cgt aag gcg ctc tca aag ttt tgc tgc ttc    1446
   His Cys Arg Thr Ala Ile Arg Lys Ala Leu Ser Lys Phe Cys Cys Phe
           460                 465                 470
   atg aag tgg ctt ggt cag gag tgc acc tgc ttc ctt cag cct gca gaa    1494
   Met Lys Trp Leu Gly Gln Glu Cys Thr Cys Phe Leu Gln Pro Ala Glu
       475                 480                 485
   ggc gcc gtc ggc gac cag ggt cat gat aat gaa gcc tat gag ggg tcc    1542
   Gly Ala Val Gly Asp Gln Gly His Asp Asn Glu Ala Tyr Glu Gly Ser
   490                 495                 500                 505
   gat gtt gac cct gct gag tcc gcc att agt gac ata tct ggg tcc tat    1590
   Asp Val Asp Pro Ala Glu Ser Ala Ile Ser Asp Ile Ser Gly Ser Tyr
                   510                 515                 520
   gtc gtc cct ggc act gcc ctc caa ccg ctc tac cag gcc ctc gat ctc    1638
   Val Val Pro Gly Thr Ala Leu Gln Pro Leu Tyr Gln Ala Leu Asp Leu
               525                 530                 535
   ccc gct gag att gtg gct cgc gcg ggc cgg ctg acc gcc aca gta aag    1686
   Pro Ala Glu Ile Val Ala Arg Ala Gly Arg Leu Thr Ala Thr Val Lys
           540                 545                 550
   gtc tcc cag gtc gat ggg cgg atc gat tgc gag acc ctt ctt ggt aac    1734
   Val Ser Gln Val Asp Gly Arg Ile Asp Cys Glu Thr Leu Leu Gly Asn
       555                 560                 565
   aaa acc ttt cgc acg tcg ttc gtt gac ggg gcg gtc tta gag acc aat    1782
   Lys Thr Phe Arg Thr Ser Phe Val Asp Gly Ala Val Leu Glu Thr Asn
   570                 575                 580                 585
   ggc cca gag cgc cac aat ctc tcc ttc gat gcc agt cag agc act atg    1830
   Gly Pro Glu Arg His Asn Leu Ser Phe Asp Ala Ser Gln Ser Thr Met
                   590                 595                 600
   gcc gct ggc cct ttc agt ctc acc tat gcc gcc tct gca gct ggg ctg    1878
   Ala Ala Gly Pro Phe Ser Leu Thr Tyr Ala Ala Ser Ala Ala Gly Leu
               605                 610                 615
   gag gtg cgc tat gtt gct gcc ggg ctt gac cat cgg gcg gtt ttt gcc    1926
   Glu Val Arg Tyr Val Ala Ala Gly Leu Asp His Arg Ala Val Phe Ala
           620                 625                 630
   ccc ggt gtt tca ccc cgg tca gcc ccc ggc gag gtt acc gcc ttc tgc    1974
   Pro Gly Val Ser Pro Arg Ser Ala Pro Gly Glu Val Thr Ala Phe Cys
       635                 640                 645
   tct gcc cta tac agg ttt aac cgt gag gcc cag cgc cat tcg ctg atc    2022
   Ser Ala Leu Tyr Arg Phe Asn Arg Glu Ala Gln Arg His Ser Leu Ile
   650                 655                 660                 665
   ggt aac tta tgg ttc cat cct gag gga ctc att ggc ctc ttc gcc ccg    2070
   Gly Asn Leu Trp Phe His Pro Glu Gly Leu Ile Gly Leu Phe Ala Pro
                   670                 675                 680
   ttt tcg ccc ggg cat gtt tgg gag tcg gct aat cca ttc tgt ggc gag    2118
   Phe Ser Pro Gly His Val Trp Glu Ser Ala Asn Pro Phe Cys Gly Glu
               685                 690                 695
   agc aca ctt tac acc cgt act tgg tcg gag gtt gat gcc gtc tct agt    2166
   Ser Thr Leu Tyr Thr Arg Thr Trp Ser Glu Val Asp Ala Val Ser Ser
           700                 705                 710
   cca gcc cgg cct gac tta ggt ttt atg tct gag cct tct ata cct agt    2214
   Pro Ala Arg Pro Asp Leu Gly Phe Met Ser Glu Pro Ser Ile Pro Ser
       715                 720                 725
   agg gcc gcc acg cct acc ctg gcg gcc cct cta ccc ccc cct gca ccg    2262
   Arg Ala Ala Thr Pro Thr Leu Ala Ala Pro Leu Pro Pro Pro Ala Pro
   730                 735                 740                 745
   gac cct tcc ccc cct ccc tct gcc ccg gcg ctt gct gag ccg gct tct    2310
   Asp Pro Ser Pro Pro Pro Ser Ala Pro Ala Leu Ala Glu Pro Ala Ser
                   750                 755                 760
   ggc gct acc gcc ggg gcc ccg gcc ata act cac cag acg gcc cgg cac    2358
   Gly Ala Thr Ala Gly Ala Pro Ala Ile Thr His Gln Thr Ala Arg His
               765                 770                 775
   cgc cgc ctg ctc ttc acc tac ccg gat ggc tct aag gta ttc gcc ggc    2406
   Arg Arg Leu Leu Phe Thr Tyr Pro Asp Gly Ser Lys Val Phe Ala Gly
           780                 785                 790
   tcg ctg ttc gag tcg aca tgc acg tgg ctc gtt aac gcg tct aat gtt    2454
   Ser Leu Phe Glu Ser Thr Cys Thr Trp Leu Val Asn Ala Ser Asn Val
       795                 800                 805
   gac cac cgc cct ggc ggc ggg ctt tgc cat gca ttt tac caa agg tac    2502
   Asp His Arg Pro Gly Gly Gly Leu Cys His Ala Phe Tyr Gln Arg Tyr
   810                 815                 820                 825
   ccc gcc tcc ttt gat gct gcc tct ttt gtg atg cgc gac ggc gcg gcc    2550
   Pro Ala Ser Phe Asp Ala Ala Ser Phe Val Met Arg Asp Gly Ala Ala
                   830                 835                 840
   gcg tac aca cta acc ccc cgg cca ata att cac gct gtc gcc cct gat    2598
   Ala Tyr Thr Leu Thr Pro Arg Pro Ile Ile His Ala Val Ala Pro Asp
               845                 850                 855
   tat agg ttg gaa cat aac cca aag agg ctt gag gct gct tat cgg gaa    2646
   Tyr Arg Leu Glu His Asn Pro Lys Arg Leu Glu Ala Ala Tyr Arg Glu
           860                 865                 870
   act tgc tcc cgc ctc ggc acc gct gca tac ccg ctc ctc ggg acc ggc    2694
   Thr Cys Ser Arg Leu Gly Thr Ala Ala Tyr Pro Leu Leu Gly Thr Gly
       875                 880                 885
   ata tac cag gtg ccg atc ggc ccc agt ttt gac gcc tgg gag cgg aac    2742
   Ile Tyr Gln Val Pro Ile Gly Pro Ser Phe Asp Ala Trp Glu Arg Asn
   890                 895                 900                 905
   cac cgc ccc ggg gat gag ttg tac ctt cct gag ctt gct gcc aga tgg    2790
   His Arg Pro Gly Asp Glu Leu Tyr Leu Pro Glu Leu Ala Ala Arg Trp
                   910                 915                 920
   ttt gag gcc aat agg ccg acc cgc ccg act ctc act ata act gag gat    2838
   Phe Glu Ala Asn Arg Pro Thr Arg Pro Thr Leu Thr Ile Thr Glu Asp
               925                 930                 935
   gtt gca cgg aca gcg aat ctg gcc atc gag ctt gac tca gcc aca gat    2886
   Val Ala Arg Thr Ala Asn Leu Ala Ile Glu Leu Asp Ser Ala Thr Asp
           940                 945                 950
   gtc ggc cgg gcc tgt gcc ggc tgt cgg gtc acc ccc ggc gtt gtt cag    2934
   Val Gly Arg Ala Cys Ala Gly Cys Arg Val Thr Pro Gly Val Val Gln
       955                 960                 965
   tac cag ttt act gca ggt gtg cct gga tcc ggc aag tcc cgc tct atc    2982
   Tyr Gln Phe Thr Ala Gly Val Pro Gly Ser Gly Lys Ser Arg Ser Ile
   970                 975                 980                 985
   acc caa gcc gat gtg gac gtt gtc gtg gtc ccg acg cgt gag ttg cgt    3030
   Thr Gln Ala Asp Val Asp Val Val Val Val Pro Thr Arg Glu Leu Arg
                   990                 995                1000
   aat gcc tgg cgc cgt cgc ggc ttt gct gct ttt acc ccg cat act gcc    3078
   Asn Ala Trp Arg Arg Arg Gly Phe Ala Ala Phe Thr Pro His Thr Ala
              1005                1010                1015
   gcc aga gtc acc cag ggg cgc cgg gtt gtc att gat gag gct cca tcc    3126
   Ala Arg Val Thr Gln Gly Arg Arg Val Val Ile Asp Glu Ala Pro Ser
          1020                1025                1030
   ctc ccc cct cac ctg ctg ctg ctc cac atg cag cgg gcc gcc acc gtc    3174
   Leu Pro Pro His Leu Leu Leu Leu His Met Gln Arg Ala Ala Thr Val
      1035                1040                1045
   cac ctt ctt ggc gac ccg aac cag atc cca gcc atc gac ttt gag cac    3222
   His Leu Leu Gly Asp Pro Asn Gln Ile Pro Ala Ile Asp Phe Glu His
   1050                1055                1060                1065
   gct ggg ctc gtc ccc gcc atc agg ccc gac tta ggc ccc acc tcc tgg    3270
   Ala Gly Leu Val Pro Ala Ile Arg Pro Asp Leu Gly Pro Thr Ser Trp
                  1070                1075                1080
   tgg cat gtt acc cat cgc tgg cct gcg gat gta tgc gag ctc atc cgt    3318
   Trp His Val Thr His Arg Trp Pro Ala Asp Val Cys Glu Leu Ile Arg
              1085                1090                1095
   ggt gca tac ccc atg atc cag acc act agc cgg gtt ctc cgt tcg ttg    3366
   Gly Ala Tyr Pro Met Ile Gln Thr Thr Ser Arg Val Leu Arg Ser Leu
           1100                1105                1110
   ttc tgg ggt gag cct gcc gtc ggg cag aaa cta gtg ttc acc cag gcg    3414
   Phe Trp Gly Glu Pro Ala Val Gly Gln Lys Leu Val Phe Thr Gln Ala
       1115                1120                1125
   gcc aag ccc gcc aac ccc ggc tca gtg acg gtc cac gag gcg cag ggc    3462
   Ala Lys Pro Ala Asn Pro Gly Ser Val Thr Val His Glu Ala Gln Gly
   1130                1135                1140                1145
   gct acc tac acg gag acc act att att gcc aca gca gat gcc cgg ggc    3510
   Ala Thr Tyr Thr Glu Thr Thr Ile Ile Ala Thr Ala Asp Ala Arg Gly
                   1150                1155                1160
   ctt att cag tcg tct cgg gct cat gcc att gtt gct ctg acg cgc cac    3558
   Leu Ile Gln Ser Ser Arg Ala His Ala Ile Val Ala Leu Thr Arg His
               1165                1170                1175
   act gag aag tgc gtc atc att gac gca cca ggc ctg ctt cgc gag gtg    3606
   Thr Glu Lys Cys Val Ile Ile Asp Ala Pro Gly Leu Leu Arg Glu Val
           1180                1185                1190
   ggc atc tcc gat gca atc gtt aat aac ttt ttc ctc gct ggt ggc gaa    3654
   Gly Ile Ser Asp Ala Ile Val Asn Asn Phe Phe Leu Ala Gly Gly Glu
       1195                1200                1205
   att ggt cac cag cgc cca tca gtt att ccc cgt ggc aac cct gac gcc    3702
   Ile Gly His Gln Arg Pro Ser Val Ile Pro Arg Gly Asn Pro Asp Ala
   1210                1215                1220                1225
   aat gtt gac acc ctg gct gcc ttc ccg ccg tct tgc cag att agt gcc    3750
   Asn Val Asp Thr Leu Ala Ala Phe Pro Pro Ser Cys Gln Ile Ser Ala
                   1230                1235                1240
   ttc cat cag ttg gct gag gag ctt ggc cac aga cct gtc cct gtt gca    3798
   Phe His Gln Leu Ala Glu Glu Leu Gly His Arg Pro Val Pro Val Ala
               1245                1250                1255
   gct gtt cta cca ccc tgc ccc gag ctc gaa cag ggc ctt ctc tac ctg    3846
   Ala Val Leu Pro Pro Cys Pro Glu Leu Glu Gln Gly Leu Leu Tyr Leu
           1260                1265                1270
   ccc cag gag ctc acc acc tgt gat agt gtc gta aca ttt gaa tta aca    3894
   Pro Gln Glu Leu Thr Thr Cys Asp Ser Val Val Thr Phe Glu Leu Thr
       1275                1280                1285
   gac att gtg cac tgc cgc atg gcc gcc ccg agc cag cgc aag gcc gtg    3942
   Asp Ile Val His Cys Arg Met Ala Ala Pro Ser Gln Arg Lys Ala Val
   1290                1295                1300                1305
   ctg tcc aca ctc gtg ggc cgc tac ggc ggt cgc aca aag ctc tac aat    3990
   Leu Ser Thr Leu Val Gly Arg Tyr Gly Gly Arg Thr Lys Leu Tyr Asn
                   1310                1315                1320
   gct tcc cac tct gat gtt cgc gac tct ctc gcc cgt ttt atc ccg gcc    4038
   Ala Ser His Ser Asp Val Arg Asp Ser Leu Ala Arg Phe Ile Pro Ala
               1325                1330                1335
   att ggc ccc gta cag gtt aca act tgt gaa ttg tac gag cta gtg gag    4086
   Ile Gly Pro Val Gln Val Thr Thr Cys Glu Leu Tyr Glu Leu Val Glu
           1340                1345                1350
   gcc atg gtc gag aag ggc cag gat ggc tcc gcc gtc ctt gag ctt gat    4134
   Ala Met Val Glu Lys Gly Gln Asp Gly Ser Ala Val Leu Glu Leu Asp
       1355                1360                1365
   ctt tgc aac cgt gac gtg tcc agg atc acc ttc ttc cag aaa gat tgt    4182
   Leu Cys Asn Arg Asp Val Ser Arg Ile Thr Phe Phe Gln Lys Asp Cys
   1370                1375                1380                1385
   aac aag ttc acc aca ggt gag acc att gcc cat ggt aaa gtg ggc cag    4230
   Asn Lys Phe Thr Thr Gly Glu Thr Ile Ala His Gly Lys Val Gly Gln
                   1390                1395                1400
   ggc atc tcg gcc tgg agc aag acc ttc tgc gcc ctc ttt ggc cct tgg    4278
   Gly Ile Ser Ala Trp Ser Lys Thr Phe Cys Ala Leu Phe Gly Pro Trp
               1405                1410                1415
   ttc cgc gct att gag aag gct att ctg gcc ctg ctc cct cag ggt gtg    4326
   Phe Arg Ala Ile Glu Lys Ala Ile Leu Ala Leu Leu Pro Gln Gly Val
           1420                1425                1430
   ttt tac ggt gat gcc ttt gat gac acc gtc ttc tcg gcg gct gtg gcc    4374
   Phe Tyr Gly Asp Ala Phe Asp Asp Thr Val Phe Ser Ala Ala Val Ala
       1435                1440                1445
   gca gca aag gca tcc atg gtg ttt gag aat gac ttt tct gag ttt gac    4422
   Ala Ala Lys Ala Ser Met Val Phe Glu Asn Asp Phe Ser Glu Phe Asp
   1450                1455                1460                1465
   tcc acc cag aat aac ttt tct ctg ggt cta gag tgt gct att atg gag    4470
   Ser Thr Gln Asn Asn Phe Ser Leu Gly Leu Glu Cys Ala Ile Met Glu
                   1470                1475                1480
   gag tgt ggg atg ccg cag tgg ctc atc cgc ctg tat cac ctt ata agg    4518
   Glu Cys Gly Met Pro Gln Trp Leu Ile Arg Leu Tyr His Leu Ile Arg
               1485                1490                1495
   tct gcg tgg atc ttg cag gcc ccg aag gag tct ctg cga ggg ttt tgg    4566
   Ser Ala Trp Ile Leu Gln Ala Pro Lys Glu Ser Leu Arg Gly Phe Trp
           1500                1505                1510
   aag aaa cac tcc ggt gag ccc ggc act ctt cta tgg aat act gtc tgg    4614
   Lys Lys His Ser Gly Glu Pro Gly Thr Leu Leu Trp Asn Thr Val Trp
       1515                1520                1525
   aat atg gcc gtt att acc cac tgt tat gac ttc cgc gat ttt cag gtg    4662
   Asn Met Ala Val Ile Thr His Cys Tyr Asp Phe Arg Asp Phe Gln Val
   1530                1535                1540                1545
   gct gcc ttt aaa ggt gat gat tcg ata gtg ctt tgc agt gag tat cgt    4710
   Ala Ala Phe Lys Gly Asp Asp Ser Ile Val Leu Cys Ser Glu Tyr Arg
                   1550                1555                1560
   cag agt cca gga gct gct gtc ctg atc gcc ggc tgt ggc ttg aag ttg    4758
   Gln Ser Pro Gly Ala Ala Val Leu Ile Ala Gly Cys Gly Leu Lys Leu
               1565                1570                1575
   aag gta gat ttc cgc ccg atc ggt ttg tat gca ggt gtt gtg gtg gcc    4806
   Lys Val Asp Phe Arg Pro Ile Gly Leu Tyr Ala Gly Val Val Val Ala
   1580 1585 1590
   ccc ggc ctt ggc gcg ctc cct gat gtt gtg cgc ttc gcc ggc cgg ctt 4854
   Pro Gly Leu Gly Ala Leu Pro Asp Val Val Arg Phe Ala Gly Arg Leu
   1595 1600 1605
   acc gag aag aat tgg ggc cct ggc cct gag cgg gcg gag cag ctc cgc 4902
   Thr Glu Lys Asn Trp Gly Pro Gly Pro Glu Arg Ala Glu Gln Leu Arg
   1610 1615 1620 1625
   ctc gct gtt agt gat ttc ctc cgc aag ctc acg aat gta gct cag atg 4950
   Leu Ala Val Ser Asp Phe Leu Arg Lys Leu Thr Asn Val Ala Gln Met
   1630 1635 1640
   tgt gtg gat gtt gtt tcc cgt gtt tat ggg gtt tcc cct gga ctc gtt 4998
   Cys Val Asp Val Val Ser Arg Val Tyr Gly Val Ser Pro Gly Leu Val
   1645 1650 1655
   cat aac ctg att ggc atg cta cag gct gtt gct gat ggc aag gca cat 5046
   His Asn Leu Ile Gly Met Leu Gln Ala Val Ala Asp Gly Lys Ala His
   1660 1665 1670
   ttc act gag tca gta aaa cca gtg ctc gac ttg aca aat tca atc ttg 5094
   Phe Thr Glu Ser Val Lys Pro Val Leu Asp Leu Thr Asn Ser Ile Leu
   1675 1680 1685
   tgt cgg gtg gaa tgaataacat gtcttttgct gcgcccatgg gttcgcgacc        5146
   Cys Arg Val Glu
   1690
   atgcgccctc ggcctatttt gttgctgctc ctcatgtttt tgcctatgct gcccgcgcca 5206
   ccgcccggtc agccgtctgg ccgccgtcgt gggcggcgca gcggcggttc cggcggtggt 5266
   ttctggggtg accgggttga ttctcagccc ttcgcaatcc cctatattca tccaaccaac 5326
   cccttcgccc ccgatgtcac cgctgcggcc ggggctggac ctcgtgttcg ccaacccgcc 5386
   cgaccactcg gctccgcttg gcgtgaccag gcccagcgcc ccgccgttgc ctcacgtcgt 5446
   agacctacca cagctggggc cgcgccgcta accgcggtcg ctccggccca tgacaccccg 5506
   ccagtgcctg atgtcgactc ccgcggcgcc atcttgcgcc ggcagtataa cctatcaaca 5566
   tctcccctta cctcttccgt ggccaccggc actaacctgg ttctttatgc cgcccctctt 5626
   agtccgcttt taccccttca ggacggcacc aatacccata taatggccac ggaagcttct 5686
   aattatgccc agtaccgggt tgcccgtgcc acaatccgtt accgcccgct ggtccccaat 5746
   gctgtcggcg gttacgccat ctccatctca ttctggccac agaccaccac caccccgacg 5806
   tccgttgata tgaattcaat aacctcgacg gatgttcgta ttttagtcca gcccggcata 5866
   gcctctgagc ttgtgatccc aagtgagcgc ctacactatc gtaaccaagg ctggcgctcc 5926
   gtcgagacct ctggggtggc tgaggaggag gctacctctg gtcttgttat gctttgcata 5986
   catggctcac tcgtaaattc ctatactaat acaccctata ccggtgccct cgggctgttg 6046
   gactttgccc ttgagcttga gtttcgcaac cttacccccg gtaacaccaa tacgcgggtc 6106
   tcccgttatt ccagcactgc tcgccaccgc cttcgtcgcg gtgcggacgg gactgccgag 6166
   ctcaccacca cggctgctac ccgctttatg aaggacctct attttactag tactaatggt 6226
   gtcggtgaga tcggccgcgg gatagccctc accctgttca accttgctga cactctgctt 6286
   ggcggcctgc cgacagaatt gatttcgtcg gctggtggcc agctgttcta ctcccgtccc 6346
   gttgtctcag ccaatggcga gccgactgtt aagttgtata catctgtaga gaatgctcag 6406
   caggataagg gtattgcaat cccgcatgac attgacctcg gagaatctcg tgtggttatt 6466
   caggattatg ataaccaaca tgaacaagat cggccgacgc cttctccagc cccatcgcgc 6526
   cctttctctg tccttcgagc taatgatgtg ctttggctct ctctcaccgc tgccgagtat 6586
   gaccagtcca cttatggctc ttcgactggc ccagtttatg tttctgactc tgtgaccttg 6646
   gttaatgttg cgaccggcgc gcaggccgtt gcccggtcgc tcgattggac caaggtcaca 6706
   cttgacggtc gccccctctc caccatccag cagtactcga agaccttctt tgtcctgccg 6766
   ctccgcggta agctctcttt ctgggaggca ggcacaacta aagccgggta cccttataat 6826
   tataacacca ctgctagcga ccaactgctt gtcgagaatg ccgccgggca ccgggtcgct 6886
   atttccactt acaccactag cctgggtgct ggtcccgtct ccatttctgc ggttgccgtt 6946
   ttagcccccc actctgcgct agcattgctt gaggatacct tggactaccc tgcccgcgcc 7006
   catacttttg atgatttctg cccagagtgc cgcccccttg gccttcaggg ctgcgctttc 7066
   cagtctactg tcgctgagct tcagcgcctt aagatgaagg tgggtaaaac tcgggagttg 7126
   tagtttattt gcttgtgccc cccttctttc tgttgcttat ttctcatttc tgcgttccgc 7186
   gctccctga 7195
<210> 7
   <211> 1693
   <212> PRT
   <213> Hepatitis E virus
<400> 7
   Met Glu Ala His Gln Phe Ile Lys Ala Pro Gly Ile Thr Thr Ala Ile
    1               5                  10                  15
   Glu Gln Ala Ala Leu Ala Ala Ala Asn Ser Ala Leu Ala Asn Ala Val
               20                  25                  30
   Val Val Arg Pro Phe Leu Ser His Gln Gln Ile Glu Ile Leu Ile Asn
           35                  40                  45
   Leu Met Gln Pro Arg Gln Leu Val Phe Arg Pro Glu Val Phe Trp Asn
       50                  55                  60
   His Pro Ile Gln Arg Val Ile His Asn Glu Leu Glu Leu Tyr Cys Arg
    65                 70                  75                  80
   Ala Arg Ser Gly Arg Cys Leu Glu Ile Gly Ala His Pro Arg Ser Ile
   85 90 95
   Asn Asp Asn Pro Asn Val Val His Arg Cys Phe Leu Arg Pro Val Gly
   100 105 110
   Arg Asp Val Gln Arg Trp Tyr Thr Ala Pro Thr Arg Gly Pro Ala Ala
   115 120 125
   Asn Cys Arg Arg Ser Ala Leu Arg Gly Leu Pro Ala Ala Asp Arg Thr
   130 135 140
   Tyr Cys Leu Asp Gly Phe Ser Gly Cys Asn Phe Pro Ala Glu Thr Gly
   145 150 155 160
   Ile Ala Leu Tyr Ser Leu His Asp Met Ser Pro Ser Asp Val Ala Glu
   165 170 175
   Ala Met Phe Arg His Gly Met Thr Arg Leu Tyr Ala Ala Leu His Leu
   180 185 190
   Pro Pro Glu Val Leu Leu Pro Pro Gly Thr Tyr Arg Thr Ala Ser Tyr
   195 200 205
   Leu Leu Ile His Asp Gly Arg Arg Val Val Val Thr Tyr Glu Gly Asp
   210 215 220
   Thr Ser Ala Gly Tyr Asn His Asp Val Ser Asn Leu Arg Ser Trp Ile
   225 230 235 240
   Arg Thr Thr Lys Val Thr Gly Asp His Pro Leu Val Ile Glu Arg Val
   245 250 255
   Arg Ala Ile Gly Cys His Phe Val Leu Leu Leu Thr Ala Ala Pro Glu
   260 265 270
   Pro Ser Pro Met Pro Tyr Val Pro Tyr Pro Arg Ser Thr Glu Val Tyr
   275 280 285
   Val Arg Ser Ile Phe Gly Pro Gly Gly Thr Pro Ser Leu Phe Pro Thr
   290 295 300
   Ser Cys Ser Thr Lys Ser Thr Phe His Ala Val Pro Ala His Ile Trp
   305 310 315 320
   Asp Arg Leu Met Leu Phe Gly Ala Thr Leu Asp Asp Gln Ala Phe Cys
   325 330 335
   Cys Ser Arg Leu Met Thr Tyr Leu Arg Gly Ile Ser Tyr Lys Val Thr
   340 345 350
   Val Gly Thr Leu Val Ala Asn Glu Gly Trp Asn Ala Ser Glu Asp Ala
   355 360 365
   Leu Thr Ala Val Ile Thr Ala Ala Tyr Leu Thr Ile Cys His Gln Arg
   370 375 380
   Tyr Leu Arg Thr Gln Ala Ile Ser Lys Gly Met Arg Arg Leu Glu Arg
   385 390 395 400
   Glu His Ala Gln Lys Phe Ile Thr Arg Leu Tyr Ser Trp Leu Phe Glu
                   405                 410                 415
   Lys Ser Gly Arg Asp Tyr Ile Pro Gly Arg Gln Leu Glu Phe Tyr Ala
               420                 425                 430
   Gln Cys Arg Arg Trp Leu Ser Ala Gly Phe His Leu Asp Pro Arg Val
           435                 440                 445
   Leu Val Phe Asp Glu Ser Ala Pro Cys His Cys Arg Thr Ala Ile Arg
       450                 455                 460
   Lys Ala Leu Ser Lys Phe Cys Cys Phe Met Lys Trp Leu Gly Gln Glu
   465                 470                 475                 480
   Cys Thr Cys Phe Leu Gln Pro Ala Glu Gly Ala Val Gly Asp Gln Gly
                   485                 490                 495
   His Asp Asn Glu Ala Tyr Glu Gly Ser Asp Val Asp Pro Ala Glu Ser
               500                 505                 510
   Ala Ile Ser Asp Ile Ser Gly Ser Tyr Val Val Pro Gly Thr Ala Leu
           515                 520                 525
   Gln Pro Leu Tyr Gln Ala Leu Asp Leu Pro Ala Glu Ile Val Ala Arg
       530                 535                 540
   Ala Gly Arg Leu Thr Ala Thr Val Lys Val Ser Gln Val Asp Gly Arg
   545                 550                 555                 560
   Ile Asp Cys Glu Thr Leu Leu Gly Asn Lys Thr Phe Arg Thr Ser Phe
                   565                 570                 575
   Val Asp Gly Ala Val Leu Glu Thr Asn Gly Pro Glu Arg His Asn Leu
               580                 585                 590
   Ser Phe Asp Ala Ser Gln Ser Thr Met Ala Ala Gly Pro Phe Ser Leu
           595                 600                 605
   Thr Tyr Ala Ala Ser Ala Ala Gly Leu Glu Val Arg Tyr Val Ala Ala
       610                 615                 620
   Gly Leu Asp His Arg Ala Val Phe Ala Pro Gly Val Ser Pro Arg Ser
   625                 630                 635                 640
   Ala Pro Gly Glu Val Thr Ala Phe Cys Ser Ala Leu Tyr Arg Phe Asn
                   645                 650                 655
   Arg Glu Ala Gln Arg His Ser Leu Ile Gly Asn Leu Trp Phe His Pro
               660                 665                 670
   Glu Gly Leu Ile Gly Leu Phe Ala Pro Phe Ser Pro Gly His Val Trp
           675                 680                 685
   Glu Ser Ala Asn Pro Phe Cys Gly Glu Ser Thr Leu Tyr Thr Arg Thr
       690                 695                 700
   Trp Ser Glu Val Asp Ala Val Ser Ser Pro Ala Arg Pro Asp Leu Gly
   705                 710                 715                 720
   Phe Met Ser Glu Pro Ser Ile Pro Ser Arg Ala Ala Thr Pro Thr Leu
   725 730 735
   Ala Ala Pro Leu Pro Pro Pro Ala Pro Asp Pro Ser Pro Pro Pro Ser
   740 745 750
   Ala Pro Ala Leu Ala Glu Pro Ala Ser Gly Ala Thr Ala Gly Ala Pro
   755 760 765
   Ala Ile Thr His Gln Thr Ala Arg His Arg Arg Leu Leu Phe Thr Tyr
   770 775 780
   Pro Asp Gly Ser Lys Val Phe Ala Gly Ser Leu Phe Glu Ser Thr Cys
   785 790 795 800
   Thr Trp Leu Val Asn Ala Ser Asn Val Asp His Arg Pro Gly Gly Gly
   805 810 815
   Leu Cys His Ala Phe Tyr Gln Arg Tyr Pro Ala Ser Phe Asp Ala Ala
   820 825 830
   Ser Phe Val Met Arg Asp Gly Ala Ala Ala Tyr Thr Leu Thr Pro Arg
   835 840 845
   Pro Ile Ile His Ala Val Ala Pro Asp Tyr Arg Leu Glu His Asn Pro
   850 855 860
   Lys Arg Leu Glu Ala Ala Tyr Arg Glu Thr Cys Ser Arg Leu Gly Thr
   865 870 875 880
   Ala Ala Tyr Pro Leu Leu Gly Thr Gly Ile Tyr Gln Val Pro Ile Gly
   885 890 895
   Pro Ser Phe Asp Ala Trp Glu Arg Asn His Arg Pro Gly Asp Glu Leu
   900 905 910
   Tyr Leu Pro Glu Leu Ala Ala Arg Trp Phe Glu Ala Asn Arg Pro Thr
   915 920 925
   Arg Pro Thr Leu Thr Ile Thr Glu Asp Val Ala Arg Thr Ala Asn Leu
   930 935 940
   Ala Ile Glu Leu Asp Ser Ala Thr Asp Val Gly Arg Ala Cys Ala Gly
   945 950 955 960
   Cys Arg Val Thr Pro Gly Val Val Gln Tyr Gln Phe Thr Ala Gly Val
   965 970 975
   Pro Gly Ser Gly Lys Ser Arg Ser Ile Thr Gln Ala Asp Val Asp Val
   980 985 990
   Val Val Val Pro Thr Arg Glu Leu Arg Asn Ala Trp Arg Arg Arg Gly
   995 1000 1005
   Phe Ala Ala Phe Thr Pro His Thr Ala Ala Arg Val Thr Gln Gly Arg
   1010 1015 1020
   Arg Val Val Ile Asp Glu Ala Pro Ser Leu Pro Pro His Leu Leu Leu
   1025 1030 1035 1040
   Leu His Met Gln Arg Ala Ala Thr Val His Leu Leu Gly Asp Pro Asn
   1045 1050 1055
   Gln Ile Pro Ala Ile Asp Phe Glu His Ala Gly Leu Val Pro Ala Ile
   1060 1065 1070
   Arg Pro Asp Leu Gly Pro Thr Ser Trp Trp His Val Thr His Arg Trp
   1075 1080 1085
   Pro Ala Asp Val Cys Glu Leu Ile Arg Gly Ala Tyr Pro Met Ile Gln
   1090 1095 1100
   Thr Thr Ser Arg Val Leu Arg Ser Leu Phe Trp Gly Glu Pro Ala Val
   1105 1110 1115 1120
   Gly Gln Lys Leu Val Phe Thr Gln Ala Ala Lys Pro Ala Asn Pro Gly
   1125 1130 1135
   Ser Val Thr Val His Glu Ala Gln Gly Ala Thr Tyr Thr Glu Thr Thr
   1140 1145 1150
   Ile Ile Ala Thr Ala Asp Ala Arg Gly Leu Ile Gln Ser Ser Arg Ala
   1155 1160 1165
   His Ala Ile Val Ala Leu Thr Arg His Thr Glu Lys Cys Val Ile Ile
   1170 1175 1180
   Asp Ala Pro Gly Leu Leu Arg Glu Val Gly Ile Ser Asp Ala Ile Val
   1185 1190 1195 1200
   Asn Asn Phe Phe Leu Ala Gly Gly Glu Ile Gly His Gln Arg Pro Ser
   1205 1210 1215
   Val Ile Pro Arg Gly Asn Pro Asp Ala Asn Val Asp Thr Leu Ala Ala
   1220 1225 1230
   Phe Pro Pro Ser Cys Gln Ile Ser Ala Phe His Gln Leu Ala Glu Glu
   1235 1240 1245
   Leu Gly His Arg Pro Val Pro Val Ala Ala Val Leu Pro Pro Cys Pro
   1250 1255 1260
   Glu Leu Glu Gln Gly Leu Leu Tyr Leu Pro Gln Glu Leu Thr Thr Cys
   1265 1270 1275 1280
   Asp Ser Val Val Thr Phe Glu Leu Thr Asp Ile Val His Cys Arg Met
   1285 1290 1295
   Ala Ala Pro Ser Gln Arg Lys Ala Val Leu Ser Thr Leu Val Gly Arg
   1300 1305 1310
   Tyr Gly Gly Arg Thr Lys Leu Tyr Asn Ala Ser His Ser Asp Val Arg
   1315 1320 1325
   Asp Ser Leu Ala Arg Phe Ile Pro Ala Ile Gly Pro Val Gln Val Thr
   1330 1335 1340
   Thr Cys Glu Leu Tyr Glu Leu Val Glu Ala Met Val Glu Lys Gly Gln
   1345 1350 1355 1360
   Asp Gly Ser Ala Val Leu Glu Leu Asp Leu Cys Asn Arg Asp Val Ser
   1365 1370 1375
   Arg Ile Thr Phe Phe Gln Lys Asp Cys Asn Lys Phe Thr Thr Gly Glu
             1380                1385                1390
   Thr Ile Ala His Gly Lys Val Gly Gln Gly Ile Ser Ala Trp Ser Lys
         1395                1400                1405
   Thr Phe Cys Ala Leu Phe Gly Pro Trp Phe Arg Ala Ile Glu Lys Ala
      1410                1415                1420
   Ile Leu Ala Leu Leu Pro Gln Gly Val Phe Tyr Gly Asp Ala Phe Asp
   1425                1430                1435                1440
   Asp Thr Val Phe Ser Ala Ala Val Ala Ala Ala Lys Ala Ser Met Val
                 1445                1450                1455
   Phe Glu Asn Asp Phe Ser Glu Phe Asp Ser Thr Gln Asn Asn Phe Ser
             1460                1465                1470
   Leu Gly Leu Glu Cys Ala Ile Met Glu Glu Cys Gly Met Pro Gln Trp
         1475                1480                1485
   Leu Ile Arg Leu Tyr His Leu Ile Arg Ser Ala Trp Ile Leu Gln Ala
      1490                1495                1500
   Pro Lys Glu Ser Leu Arg Gly Phe Trp Lys Lys His Ser Gly Glu Pro
   1505                1510                1515                1520
   Gly Thr Leu Leu Trp Asn Thr Val Trp Asn Met Ala Val Ile Thr His
                 1525                1530                1535
   Cys Tyr Asp Phe Arg Asp Phe Gln Val Ala Ala Phe Lys Gly Asp Asp
             1540                1545                1550
   Ser Ile Val Leu Cys Ser Glu Tyr Arg Gln Ser Pro Gly Ala Ala Val
         1555                1560                1565
   Leu Ile Ala Gly Cys Gly Leu Lys Leu Lys Val Asp Phe Arg Pro Ile
      1570                1575                1580
   Gly Leu Tyr Ala Gly Val Val Val Ala Pro Gly Leu Gly Ala Leu Pro
   1585                1590                1595                1600
   Asp Val Val Arg Phe Ala Gly Arg Leu Thr Glu Lys Asn Trp Gly Pro
                 1605                1610                1615
   Gly Pro Glu Arg Ala Glu Gln Leu Arg Leu Ala Val Ser Asp Phe Leu
             1620                1625                1630
   Arg Lys Leu Thr Asn Val Ala Gln Met Cys Val Asp Val Val Ser Arg
         1635                1640                1645
   Val Tyr Gly Val Ser Pro Gly Leu Val His Asn Leu Ile Gly Met Leu
      1650                1655                1660
   Gln Ala Val Ala Asp Gly Lys Ala His Phe Thr Glu Ser Val Lys Pro
   1665                1670                1675                1680
   Val Leu Asp Leu Thr Asn Ser Ile Leu Cys Arg Val Glu
                 1685                1690
<210> 8
   <211> 660
   <212> PRT
   <213> Hepatitis E virus
<400> 8
   Met Arg Pro Arg Pro Ile Leu Leu Leu Leu Leu Met Phe Leu Pro Met
    1               5                  10                  15
   Leu Pro Ala Pro Pro Pro Gly Gln Pro Ser Gly Arg Arg Arg Gly Arg
               20                  25                  30
   Arg Ser Gly Gly Ser Gly Gly Gly Phe Trp Gly Asp Arg Val Asp Ser
           35                  40                  45
   Gln Pro Phe Ala Ile Pro Tyr Ile His Pro Thr Asn Pro Phe Ala Pro
       50                  55                  60
   Asp Val Thr Ala Ala Ala Gly Ala Gly Pro Arg Val Arg Gln Pro Ala
   65                  70                  75                  80
   Arg Pro Leu Gly Ser Ala Trp Arg Asp Gln Ala Gln Arg Pro Ala Val
                   85                  90                  95
   Ala Ser Arg Arg Arg Pro Thr Thr Ala Gly Ala Ala Pro Leu Thr Ala
               100                 105                 110
   Val Ala Pro Ala His Asp Thr Pro Pro Val Pro Asp Val Asp Ser Arg
           115                 120                 125
   Gly Ala Ile Leu Arg Arg Gln Tyr Asn Leu Ser Thr Ser Pro Leu Thr
       130                 135                 140
   Ser Ser Val Ala Thr Gly Thr Asn Leu Val Leu Tyr Ala Ala Pro Leu
   145                 150                 155                 160
   Ser Pro Leu Leu Pro Leu Gln Asp Gly Thr Asn Thr His Ile Met Ala
                   165                 170                 175
   Thr Glu Ala Ser Asn Tyr Ala Gln Tyr Arg Val Ala Arg Ala Thr Ile
               180                 185                 190
   Arg Tyr Arg Pro Leu Val Pro Asn Ala Val Gly Gly Tyr Ala Ile Ser
           195                 200                 205
   Ile Ser Phe Trp Pro Gln Thr Thr Thr Thr Pro Thr Ser Val Asp Met
       210                 215                 220
   Asn Ser Ile Thr Ser Thr Asp Val Arg Ile Leu Val Gln Pro Gly Ile
   225                 230                 235                 240
   Ala Ser Glu Leu Val Ile Pro Ser Glu Arg Leu His Tyr Arg Asn Gln
                   245                 250                 255
   Gly Trp Arg Ser Val Glu Thr Ser Gly Val Ala Glu Glu Glu Ala Thr
               260                 265                 270
   Ser Gly Leu Val Met Leu Cys Ile His Gly Ser Leu Val Asn Ser Tyr
           275                 280                 285
   Thr Asn Thr Pro Tyr Thr Gly Ala Leu Gly Leu Leu Asp Phe Ala Leu
   290 295 300
   Glu Leu Glu Phe Arg Asn Leu Thr Pro Gly Asn Thr Asn Thr Arg Val
   305 310 315 320
   Ser Arg Tyr Ser Ser Thr Ala Arg His Arg Leu Arg Arg Gly Ala Asp
   325 330 335
   Gly Thr Ala Glu Leu Thr Thr Thr Ala Ala Thr Arg Phe Met Lys Asp
   340 345 350
   Leu Tyr Phe Thr Ser Thr Asn Gly Val Gly Glu Ile Gly Arg Gly Ile
   355 360 365
   Ala Leu Thr Leu Phe Asn Leu Ala Asp Thr Leu Leu Gly Gly Leu Pro
   370 375 380
   Thr Glu Leu Ile Ser Ser Ala Gly Gly Gln Leu Phe Tyr Ser Arg Pro
   385 390 395 400
   Val Val Ser Ala Asn Gly Glu Pro Thr Val Lys Leu Tyr Thr Ser Val
   405 410 415
   Glu Asn Ala Gln Gln Asp Lys Gly Ile Ala Ile Pro His Asp Ile Asp
   420 425 430
   Leu Gly Glu Ser Arg Val Val Ile Gln Asp Tyr Asp Asn Gln His Glu
   435 440 445
   Gln Asp Arg Pro Thr Pro Ser Pro Ala Pro Ser Arg Pro Phe Ser Val
   450 455 460
   Leu Arg Ala Asn Asp Val Leu Trp Leu Ser Leu Thr Ala Ala Glu Tyr
   465 470 475 480
   Asp Gln Ser Thr Tyr Gly Ser Ser Thr Gly Pro Val Tyr Val Ser Asp
   485 490 495
   Ser Val Thr Leu Val Asn Val Ala Thr Gly Ala Gln Ala Val Ala Arg
   500 505 510
   Ser Leu Asp Trp Thr Lys Val Thr Leu Asp Gly Arg Pro Leu Ser Thr
   515 520 525
   Ile Gln Gln Tyr Ser Lys Thr Phe Phe Val Leu Pro Leu Arg Gly Lys
   530 535 540
   Leu Ser Phe Trp Glu Ala Gly Thr Thr Lys Ala Gly Tyr Pro Tyr Asn
   545 550 555 560
   Tyr Asn Thr Thr Ala Ser Asp Gln Leu Leu Val Glu Asn Ala Ala Gly
   565 570 575
   His Arg Val Ala Ile Ser Thr Tyr Thr Thr Ser Leu Gly Ala Gly Pro
   580 585 590
   Val Ser Ile Ser Ala Val Ala Val Leu Ala Pro His Ser Ala Leu Ala
   595 600 605
   Leu Leu Glu Asp Thr Leu Asp Tyr Pro Ala Arg Ala His Thr Phe Asp
   610 615 620
   Asp Phe Cys Pro Glu Cys Arg Pro Leu Gly Leu Gln Gly Cys Ala Phe
   625 630 635 640
   Gln Ser Thr Val Ala Glu Leu Gln Arg Leu Lys Met Lys Val Gly Lys
   645 650 655
   Thr Arg Glu Leu
   660
<210> 9
   <211> 123
   <212> PRT
   <213> Hepatitis E virus
<400> 9
   Met Asn Asn Met Ser Phe Ala Ala Pro Met Gly Ser Arg Pro Cys Ala
   1 5 10 15
   Leu Gly Leu Phe Cys Cys Cys Ser Ser Cys Phe Cys Leu Cys Cys Pro
   20 25 30
   Arg His Arg Pro Val Ser Arg Leu Ala Ala Val Val Gly Gly Ala Ala
   35 40 45
   Ala Val Pro Ala Val Val Ser Gly Val Thr Gly Leu Ile Leu Ser Pro
   50 55 60
   Ser Gln Ser Pro Ile Phe Ile Gln Pro Thr Pro Ser Pro Pro Met Ser
   65 70 75 80
   Pro Leu Arg Pro Gly Leu Asp Leu Val Phe Ala Asn Pro Pro Asp His
   85 90 95
   Ser Ala Pro Leu Gly Val Thr Arg Pro Ser Ala Pro Pro Leu Pro His
   100 105 110
   Val Val Asp Leu Pro Gln Leu Gly Pro Arg Arg
   115 120
<210> 10
   <211> 7171
   <212> DNA
   <213> Hepatitis E virus
<400> 10
   gccatggagg cccaccagtt cattaaggct cctggcatca ctactgctat tgagcaagca 60
   gctctagcag cggccaactc cgcccttgcg aatgctgtgg tggtccggcc tttcctttcc 120
   catcagcagg ttgagatcct tataaatctc atgcaacctc ggcagctggt gtttcgtcct 180
   gaggtttttt ggaatcaccc gattcaacgt gttatacata atgagcttga gcagtattgc 240
   cgtgctcgct cgggtcgctg ccttgagatt ggagcccacc cacgctccat taatgataat 300
   cctaatgtcc tccatcgctg ctttctccac cccgtcggcc gggatgttca gcgctggtac 360
   acagccccga ctaggggacc tgcggcgaac tgtcgccgct cggcacttcg tggtctgcca 420
   ccagccgacc gcacttactg ttttgatggc tttgccggct gccgttttgc cgccgagact 480
   ggtgtggctc tctattctct ccatgacttg cagccggctg atgttgccga ggcgatggct 540
   cgccacggca tgacccgcct ttatgcagct ttccacttgc ctccagaggt gctcctgcct 600
   cctggcacct accggacatc atcctacttg ctgatccacg atggtaagcg cgcggttgtc 660
   acttatgagg gtgacactag cgccggttac aatcatgatg ttgccaccct ccgcacatgg 720
   atcaggacaa ctaaggttgt gggtgaacac cctttggtga tcgagcgggt gcggggtatt 780
   ggctgtcact ttgtgttgtt gatcactgcg gcccctgagc cctccccgat gccctacgtt 840
   ccttacccgc gttcgacgga ggtctatgtc cggtctatct ttgggcccgg cgggtccccg 900
   tcgctgttcc cgaccgcttg tgctgtcaag tccacttttc acgccgtccc cacgcacatc 960
   tgggaccgtc tcatgctctt tggggccacc ctcgacgacc aggccttttg ctgctccagg 1020
   cttatgacgt accttcgtgg cattagctat aaggtaactg tgggtgccct ggtcgctaat 1080
   gaaggctgga atgccaccga ggatgcgctc actgcagtta ttacggcggc ttacctcaca 1140
   atatgtcatc agcgttattt gcggacccag gcgatttcta agggcatgcg ccggcttgag 1200
   cttgaacatg ctcagaaatt tatttcacgc ctctacagct ggctatttga gaagtcaggt 1260
   cgtgattaca tcccaggccg ccagctgcag ttctacgctc agtgccgccg ctggttatct 1320
   gccgggttcc atctcgaccc ccgcacctta gtttttgatg agtcagtgcc ttgtagctgc 1380
   cgaaccacca tccggcggat cgctggaaaa ttttgctgtt ttatgaagtg gctcggtcag 1440
   gagtgttctt gtttcctcca gcccgccgag gggctggcgg gcgaccaagg tcatgacaat 1500
   gaggcctatg aaggctctga tgttgatact gctgagcctg ccaccctaga cattacaggc 1560
   tcatacatcg tggatggtcg gtctctgcaa actgtctatc aagctctcga cctgccagct 1620
   gacctggtag ctcgcgcagc ccgactgtct gctacagtta ctgttactga aacctctggc 1680
   cgtctggatt gccaaacaat gatcggcaat aagacttttc tcactacctt tgttgatggg 1740
   gcacgccttg aggttaacgg gcctgagcag cttaacctct cttttgacag ccagcagtgt 1800
   agtatggcag ccggcccgtt ttgcctcacc tatgctgccg tagatggcgg gctggaagtt 1860
   catttttcca ccgctggcct cgagagccgt gttgttttcc cccctggtaa tgccccgact 1920
   gccccgccga gtgaggtcac cgccttctgc tcagctcttt ataggcacaa ccggcagagc 1980
   cagcgccagt cggttattgg tagtttgtgg ctgcaccctg aaggtttgct cggcctgttc 2040
   ccgccctttt cacccgggca tgagtggcgg tctgctaacc cattttgcgg cgagagcacg 2100
   ctctacaccc gcacttggtc cacaattaca gacacaccct taactgtcgg gctaatttcc 2160
   ggtcatttgg atgctgctcc ccactcgggg gggccacctg ctactgccac aggccctgct 2220
   gtaggctcgt ctgactctcc agaccctgac ccgctacctg atgttacaga tggctcacgc 2280
   ccctctgggg cccgtccggc tggccccaac ccgaatggcg ttccgcagcg ccgcttacta 2340
   cacacctacc ctgacggcgc taagatctat gtcggctcca ttttcgagtc tgagtgcacc 2400
   tggcttgtca acgcatctaa cgccggccac cgccctggtg gcgggctttg tcatgctttt 2460
   tttcagcgtt accctgattc gtttgacgcc accaagtttg tgatgcgtga tggtcttgcc 2520
   gcgtataccc ttacaccccg gccgatcatt catgcggtgg ccccggacta tcgattggaa 2580
   cataacccca agaggctcga ggctgcctac cgcgagactt gcgcccgccg aggcactgct 2640
   gcctatccac tcttaggcgc tggcatttac caggtgcctg ttagtttgag ttttgatgcc 2700
   tgggagcgga accaccgccc gtttgacgag ctttacctaa cagagctggc ggctcggtgg 2760
   tttgaatcca accgccccgg tcagcccacg ttgaacataa ctgaggatac cgcccgtgcg 2820
   gccaacctgg ccctggagct tgactccggg agtgaagtag gccgcgcatg tgccgggtgt 2880
   aaagtcgagc ctggcgttgt gcggtatcag tttacagccg gtgtccccgg ctctggcaag 2940
   tcaaagtccg tgcaacaggc ggatgtggat gttgttgttg tgcccactcg cgagcttcgg 3000
   aacgcttggc ggcgccgggg ctttgcggca ttcactccgc acactgcggc ccgtgtcact 3060
   agcggccgta gggttgtcat tgatgaggcc ccttcgctcc ccccacactt gctgctttta 3120
   catatgcagc gtgctgcatc tgtgcacctc cttggggacc cgaatcagat ccccgccata 3180
   gattttgagc acaccggtct gattccagca atacggccgg agttggtccc gacttcatgg 3240
   tggcatgtca cccaccgttg ccctgcagat gtctgtgagt tagtccgtgg tgcttaccct 3300
   aaaatccaga ctacaagtaa ggtgctccgt tcccttttct ggggagagcc agctgtcggc 3360
   cagaagctag tgttcacaca ggctgctaag gccgcgcacc ccggatctat aacggtccat 3420
   gaggcccagg gtgccacttt taccactaca actataattg caactgcaga tgcccgtggc 3480
   ctcatacagt cctcccgggc tcacgctata gttgctctca ctaggcatac tgaaaaatgt 3540
   gttatacttg actctcccgg cctgttgcgt gaggtgggta tctcagatgc cattgttaat 3600
   aatttcttcc tttcgggtgg cgaggttggt caccagagac catcggtcat tccgcgaggc 3660
   aaccctgacc gcaatgttga cgtgcttgcg gcgtttccac cttcatgcca aataagcgcc 3720
   ttccatcagc ttgctgagga gctgggccac cggccggcgc cggtggcggc tgtgctacct 3780
   ccctgccctg agcttgagca gggccttctc tatctgccac aggagctagc ctcctgtgac 3840
   agtgttgtga catttgagct aactgacatt gtgcactgcc gcatggcggc ccctagccaa 3900
   aggaaagctg ttttgtccac gctggtaggc cggtatggca gacgcacaag gctttatgat 3960
   gcgggtcaca ccgatgtccg cgcctccctt gcgcgcttta ttcccactct cgggcgggtt 4020
   actgccacca cctgtgaact ctttgagctt gtagaggcga tggtggagaa gggccaagac 4080
   ggttcagccg tcctcgagtt ggatttgtgc agccgagatg tctcccgcat aacctttttc 4140
   cagaaggatt gtaacaagtt cacgaccggc gagacaattg cgcatggcaa agtcggtcag 4200
   ggtatcttcc gctggagtaa gacgttttgt gccctgtttg gcccctggtt ccgtgcgatt 4260
   gagaaggcta ttctatccct tttaccacaa gctgtgttct acggggatgc ttatgacgac 4320
   tcagtattct ctgctgccgt ggctggcgcc agccatgcca tggtgtttga aaatgatttt 4380
   tctgagtttg actcgactca gaataacttt tccctaggtc ttgagtgcgc cattatggaa 4440
   gagtgtggta tgccccagtg gcttgtcagg ttgtaccatg ccgtccggtc ggcgtggatc 4500
   ctgcaggccc caaaagagtc tttgagaggg ttctggaaga agcattctgg tgagccgggc 4560
   agcttgctct ggaatacggt gtggaacatg gcaatcattg cccattgcta tgagttccgg 4620
   gacctccagg ttgccgcctt caagggcgac gactcggtcg tcctctgtag tgaataccgc 4680
   cagagcccag gcgccggttc gcttatagca ggctgtggtt tgaagttgaa ggctgacttc 4740
   cggccgattg ggctgtatgc cggggttgtc gtcgccccgg ggctcggggc cctacccgat 4800
   gtcgttcgat tcgccggacg gctttcggag aagaactggg ggcctgatcc ggagcgggca 4860
   gagcagctcc gcctcgccgt gcaggatttc ctccgtaggt taacgaatgt ggcccagatt 4920
   tgtgttgagg tggtgtctag agtttacggg gtttccccgg gtctggttca taacctgata 4980
   ggcatgctcc agactattgg tgatggtaag gcgcatttta cagagtctgt taagcctata 5040
   cttgacctta cacactcaat tatgcaccgg tctgaatgaa taacatgtgg tttgctgcgc 5100
   ccatgggttc gccaccatgc gccctaggcc tcttttgctg ttgttcctct tgtttctgcc 5160
   tatgttgccc gcgccaccga ccggtcagcc gtctggccgc cgtcgtgggc ggcgcagcgg 5220
   cggtaccggc ggtggtttct ggggtgaccg ggttgattct cagcccttcg caatccccta 5280
   tattcatcca accaacccct ttgccccaga cgttgccgct gcgtccgggt ctggacctcg 5340
   ccttcgccaa ccagcccggc cacttggctc cacttggcga gatcaggccc agcgcccctc 5400
   cgctgcctcc cgtcgccgac ctgccacagc cggggctgcg gcgctgacgg ctgtggcgcc 5460
   tgcccatgac acctcacccg tcccggacgt tgattctcgc ggtgcaattc tacgccgcca 5520
   gtataatttg tctacttcac ccctgacatc ctctgtggcc tctggcacta atttagtcct 5580
   gtatgcagcc ccccttaatc cgcctctgcc gctgcaggac ggtactaata ctcacattat 5640
   ggccacagag gcctccaatt atgcacagta ccgggttgcc cgcgctacta tccgttaccg 5700
   gcccctagtg cctaatgcag ttggaggcta tgctatatcc atttctttct ggcctcaaac 5760
   aaccacaacc cctacatctg ttgacatgaa ttccattact tccactgatg tcaggattct 5820
   tgttcaacct ggcatagcat ctgaattggt catcccaagc gagcgccttc actaccgcaa 5880
   tcaaggttgg cgctcggttg agacatctgg tgttgctgag gaggaagcca cctccggtct 5940
   tgtcatgtta tgcatacatg gctctccagt taactcctat accaataccc cttataccgg 6000
   tgcccttggc ttactggact ttgccttaga gcttgagttt cgcaatctca ccacctgtaa 6060
   caccaataca cgtgtgtccc gttactccag cactgctcgt cactccgccc gagggggcga 6120
   cgggactgcg gagctgacca caactgcagc caccaggttc atgaaagatc tccactttac 6180
   cggccttaat ggggtaggtg aagtcggccg cgggatagct ctaacattac ttaaccttgc 6240
   tgacacgctc ctcggcgggc tcccgacaga attaatttcg tcggctggcg ggcaactgtt 6300
   ttattcccgc ccggttgtct cagccaatgg cgagccaacc gtgaagctct atacatcagt 6360
   ggagaatgct cagcaggata agggtgttgc tatcccccac gatatcgatc ttggtgattc 6420
   gcgtgtggtc attcaggatt atgacaacca gcatgagcag gatcggccca ccccgtcgcc 6480
   tgcgccatct cggccttttt ctgttctccg agcaaatgat gtactttggc tgtccctcac 6540
   tgcagccgag tatgaccagt ccacttacgg gtcgtcaact ggcccggttt atatctcgga 6600
   cagcgtgact tggtgaatgg ttgcgactgg cgcgcaggcc gtagcccgat cgcttgactg 6660
   gtccaaagtc accctcgacg ggcggcccct cccgactgtt gagcaatatt ccaagacatt 6720
   ctttgtgctc ccccttcgtg gcaagctctc cttttgggag gccggcacaa caaaagcagg 6780
   ttatccttat aattataata ctactgctag tgaccagatt ctgattgaaa atgctgccgg 6840
   ccatcgggtc gccatttcaa cctataccac caggcttggg gccggtccgg tcgccatttc 6900
   tgcggccgcg gttttggctc cacgctccgc cctggctctg ctggaggata cttttgatta 6960
   tccggggcgg gcgcacacat ttgatgactt ctgccctgaa tgccgcgctt taggcctcca 7020
   gggttgtgct ttccagtcaa ctgtcgctga gctccagcgc cttaaagtta aggtgggtaa 7080
   aactcgggag ttgtagttta tttggctgtg cccacctact tatatctgct gatttccttt 7140
   atttcctttt tctcggtccc gcgctccctg a                                 7171
<210> 11
   <211> 1575
   <212> DNA
   <213> Hepatitis E virus
<400> 11
   gttgcgtgag gtgggtatct cagatgccat tgttaataat ttcttccttt cgggtggcga 60
   ggttggtcac cagagaccat cggtcattcc gcgaggcaac cctgaccgca atgttgacgt 120
   gcttgcggcg tttccacctt catgccaaat aagcgccttc catcagcttg ctgaggagct 180
   gggccaccgg ccggcgccgg tggcggctgt gctacctccc tgccctgagc ttgagcaggg 240
   ccttctctat ctgccacagg agctagcctc ctgtgacagt gttgtgacat ttgagctaac 300
   tgacattgtg cactgccgca tggcggcccc tagccaaagg aaagctgttt tgtccacgct 360
   ggtaggccgg tatggcagac gcacaaggct ttatgatgcg ggtcacaccg atgtccgcgc 420
   ctcccttgcg cgctttattc ccactctcgg gcgggttact gccaccacct gtgaactctt 480
   tgagcttgta gaggcgatgg tggagaaggg ccaagacggt tcagccgtcc tcgagttgga 540
   tttgtgcagc cgagatgtct cccgcataac ctttttccag aaggattgta acaagttcac 600
   gaccggcgag acaattgcgc atggcaaagt cggtcagggt atcttccgct ggagtaagac 660
   cttttgtgcc ctgtttggcc cctggttccg tgcgattgag aaggctattc tatccctttt 720
   accacaagct gtgttctacg gggatgctta tgacgactca gtattctctg ctgccgtggc 780
   tggcgccagc catgccatgg tgtttgaaaa tgatttttct gagtttgact cgactcagaa 840
   taacttttcc ctaggtcttg agtgcgccat tatggaagag tgtggtatgc cccagtggct 900
   tgtcaggttg taccatgccg tccggtcggc gtggatcctg caggccccaa aagagtcttt 960
   gagagggttc tggaagaagc attctggtga gccgggcacg ttgctctgga atacggtgtg 1020
   gaacatggca atcattgccc attgctatga gttccgggac ctccaggttg ccgccttcaa 1080
   gggcgacgac tcggtcgtcc tctgtagtga ataccgccag agcccaggcg ccggttcgct 1140
   tatagcaggc tgtggtttga agttgaaggc tgacttccgg ccgattgggc tgtatgccgg 1200
   ggttgtcgtc gccccggggc tcggggccct acccgatgtc gttcgattcg ccggacggct 1260
   ttcggagaag aactgggggc ctgatccgga gcgggcagag cagctccgcc tcgccgtgca 1320
   ggatttcctc cgtaggttaa cgaatgtggc ccagatttgt gttgaggtgg tgtctagagt 1380
   ttacggggtt tccccgggtc tggttcataa cctgataggc atgctccaga ctattggtga 1440
   tggtaaggcg cattttacag agtctgttaa gcctatactt gaccttacac actcaattat 1500
   gcaccggtct gaatgaataa catgtggttt gctgcgccca tgggttcgcc accatgcgcc 1560
   ctaggcctct tttgc 1575
<210> 12
   <211> 874
   <212> DNA
   <213> Hepatitis E virus
<400> 12
   cgggccccgt acaggtcaca acctgtgagt tgtacgagct agtggaggcc atggtcgaga 60
   aaggccagga tggctccgcc gtccttgagc tcgatctctg caaccgtgac gtgtccagga 120
   tcaccttttt ccagaaagat tgcaataagt tcaccacggg agagaccatc gcccatggta 180
   aagtgggcca gggcatttcg gcctggagta agaccttctg tgcccttttc ggcccctggt 240
   tccgtgctat tgagaaggct attctggccc tgctccctca gggtgtgttt tatggggatg 300
   cctttgatga caccgtcttc tcggcgcgtg tggccgcagc aaaggcgtcc atggtgtttg 360
   agaatgactt ttctgagttt gactccaccc agaataattt ttccctgggc ctagagtgtg 420
   ctattatgga gaagtgtggg atgccgaagt ggctcatccg cttgtaccac cttataaggt 480
   ctgcgtggat cctgcaggcc ccgaaggagt ccctgcgagg gtgttggaag aaacactccg 540
   gtgagcccgg cactcttcta tggaatactg tctggaacat ggccgttatc acccattgtt 600
   acgatttccg cgatttgcag gtggctgcct ttaaaggtga tgattcgata gtgctttgca 660
   gtgagtaccg tcagagtcca ggggctgctg tcctgattgc tggctgtggc ttaaagctga 720
   aggtgggttt ccgtccgatt ggtttgtatg caggtgttgt ggtgaccccc ggccttggcg 780
   cgcttcccga cgtcgtgcgc ttgtccggcc ggcttactga gaagaattgg ggccctggcc 840
   ctgagcgggc ggagcagctc cgccttgctg tgcg 874
<210> 13
   <211> 449
   <212> DNA
   <213> Hepatitis E virus
<220>
   <221> CDS
   <222> (2)..(100)
<400> 13
   c gcc aac cag ccc ggc cac ttg gct cca ctt ggc gag atc agg ccc agc 49
     Ala Asn Gln Pro Gly His Leu Ala Pro Leu Gly Glu Ile Arg Pro Ser
     1               5                   10                  15
   gcc cct ccg ctg cct ccc gtc gcc gac ctg cca cag ccg ggg ctg cgg   97
   Ala Pro Pro Leu Pro Pro Val Ala Asp Leu Pro Gln Pro Gly Leu Arg
               20                  25                  30
   cgc tgacggctgt ggcgcctgcc catgacacct cacccgtccc ggacgttgat       150
   Arg
   tctcgcggtg caattctacg ccgccagtat aatttgtcta cttcacccct gacatcctct 210
   gtggcctctg gcactaattt agtcctgtat gcagcccccc ttaatccgcc tctgccgctg 270
   caggacggta ctaatactca cattatggcc acagaggcct ccaattatgc acagtaccgg 330
   gttgcccgcg ctactatccg ttaccggccc ctagtgccta atgcagttgg aggctatgct 390
   atatccattt ctttctggcc tcaaacaacc acaaccccta catctgttga catgaattc  449
<210> 14
   <211> 33
   <212> PRT.
   <213> Hepatitis E virus
<400> 14
   Ala Asn Gln Pro Gly His Leu Ala Pro Leu Gly Glu Ile Arg Pro Ser
   1               5                   10                  15
   Ala Pro Pro Leu Pro Pro Val Ala Asp Leu Pro Gln Pro Gly Leu Arg
               20                  25                  30
   Arg
<210> 15
   <211> 130
   <212> DNA
   <213> Hepatitis E virus
<220>
   <221> CDS
   <222> (5)..(130)
<400> 15
   ggat act ttt gat tat ccg ggg cgg gcg cac aca ttt gat gac ttc tgc   49
        Thr Phe Asp Tyr Pro Gly Arg Ala His Thr Phe Asp Asp Phe Cys
         1               5                  10                  15
   cct gaa tgc cgc gct tta ggc ctc cag ggt tgt gct ttc cag tca act    97
   Pro Glu Cys Arg Ala Leu Gly Leu Gln Gly Cys Ala Phe Gln Ser Thr
                   20                  25                  30
   gtc gct gag ctc cag cgc ctt aaa gtt aag gtt                        130
   Val Ala Glu Leu Gln Arg Leu Lys Val Lys Val
               35                  40
<210> 16
   <211> 42
   <212> PRT
   <213> Hepatitis E virus
<400> 16
   Thr Phe Asp Tyr Pro Gly Arg Ala His Thr Phe Asp Asp Phe Cys Pro
    1               5                  10                  15
   Glu Cys Arg Ala Leu Gly Leu Gln Gly Cys Ala Phe Gln Ser Thr Val
               20                  25                  30
   Ala Glu Leu Gln Arg Leu Lys Val Lys Val
           35                  40
<210> 17
   <211> 33
   <212> PRT
   <213> Hepatitis E virus
<400> 17
   Ala Asn Gln Pro Gly His Leu Ala Pro Leu Gly Glu Ile Arg Pro Ser
    1               5                  10                  15
   Ala Pro Pro Leu Pro Pro Val Ala Asp Leu Pro Gln Pro Gly Leu Arg
               20                  25                  30
   Arg
<210> 18
   <211> 33
   <212> PRT
   <213> Hepatitis E virus
<400> 18
   Ala Asn Pro Pro Asp His Ser Ala Pro Leu Gly Val Thr Arg Pro Ser
   1 5 10 15
   Ala Pro Pro Leu Pro His Val Val Asp Leu Pro Gln Leu Gly Pro Arg
   20 25 30
   Arg
<210> 19
   <211> 42
   <212> PRT
   <213> Hepatitis E virus
<400> 19
   Thr Phe Asp Tyr Pro Gly Arg Ala His Thr Phe Asp Asp Phe Cys Pro
   1 5 10 15
   Glu Cys Arg Ala Leu Gly Leu Gln Gly Cys Ala Phe Gln Ser Thr Val
   20 25 30
   Ala Glu Leu Gln Arg Leu Lys Val Lys Val
   35 40
<210> 20
   <211> 42
   <212> PRT
   <213> Hepatitis E virus
<400> 20
   Thr Leu Asp Tyr Pro Ala Arg Ala His Thr Phe Asp Asp Phe Cys Pro
   1 5 10 15
   Glu Cys Arg Pro Leu Gly Leu Gln Gly Cys Ala Phe Gln Ser Thr Val
   20 25 30
   Ala Glu Leu Gln Arg Leu Lys Met Lys Val
   35 40
<210> 21
   <211> 18
   <212> DNA
   <213> Artificial Sequence
<220>
   <223> Synthetic oligonucleotide
<400> 21
   ggaattcgcg gccgctcg 18
<210> 22
   <211> 20
   <212> DNA
   <213> Artificial Sequence
<220>
   <223> Synthetic oligonucleotide
<400> 22
   cgagcggccg cgaattcctt 20
<210> 23
   <211> 62
   <212> PRT
   <213> Hepatitis E virus
<400> 23
   Gln Thr Leu Cys Thr Ala Ala Trp Pro Pro Arg Ala Ser Ala Arg Pro
    1               5                  10                  15
   Cys Cys Pro His Ser Trp Ala Ala Thr Ala Val Ala Gln Ser Ser Thr
               20                  25                  30
   Met Leu Pro Thr Leu Met Phe Ala Thr Leu Ser Pro Val Leu Ser Arg
           35                  40                  45
   Pro Leu Ala Pro Tyr Arg Leu Gln Leu Val Asn Cys Thr Ser
       50                  55                  60
<210> 24
   <211> 123
   <212> PRT
   <213> Hepatitis E virus
<400> 24
   Trp Arg Pro Trp Ser Arg Arg Ala Arg Met Ala Pro Pro Ser Leu Ser
    1               5                  10                  15
   Leu Ile Phe Ala Thr Val Thr Cys Pro Gly Ser Pro Ser Ser Arg Lys
               20                  25                  30
   Ile Val Thr Ser Ser Pro Gln Val Arg Pro Leu Pro Met Val Lys Trp
           35                  40                  45
   Ala Arg Ala Ser Arg Pro Gly Ala Arg Pro Ser Ala Pro Ser Leu Ala
       50                  55                  60
   Leu Gly Ser Ala Leu Leu Arg Arg Leu Phe Trp Pro Cys Ser Leu Arg
   65                  70                  75                  80
   Val Cys Phe Thr Val Met Pro Leu Met Thr Pro Ser Ser Arg Arg Leu
                   85                  90                  95
   Trp Pro Gln Gln Arg His Pro Trp Cys Leu Arg Met Thr Phe Leu Ser
               100                 105                 110
   Leu Thr Pro Pro Arg Ile Thr Phe Leu Trp Val
           115                 120
<210> 25
   <211> 20
   <212> PRT
   <213> Hepatitis E virus
<400> 25
   Ser Val Leu Leu Trp Arg Ser Val Gly Cys Arg Ser Gly Ser Ser Ala
    1               5                  10                  15
   Cys Ile Thr Leu
               20
<210> 26
   <211> 57
   <212> PRT
   <213> Hepatitis E virus
<400> 26
   Gly Leu Arg Gly Ser Cys Arg Pro Arg Arg Ser Leu Cys Glu Gly Phe
    1               5                  10                  15
   Gly Arg Asn Thr Pro Val Ser Pro Ala Leu Phe Tyr Gly Ile Leu Ser
               20                  25                  30
   Gly Ile Trp Pro Leu Leu Pro Thr Val Met Thr Ser Ala Ile Phe Arg
           35                  40                  45
   Trp Leu Pro Leu Lys Val Met Ile Arg
       50                  55
<210> 27
   <211> 14
   <212> PRT
   <213> Hepatitis E virus
<400> 27
   Cys Phe Ala Val Ser Ile Val Arg Val Gln Glu Leu Leu Ser
    1               5                  10
<210> 28
   <211> 5
   <212> PRT
   <213> Hepatitis E virus
<400> 28
   Ser Pro Ala Val Ala
    1               5
<210> 29
   <211> 58
   <212> PRT
   <213> Hepatitis E virus
<400> 29
   Ile Ser Ala Arg Ser Val Cys Met Gln Val Leu Trp Trp Pro Pro Ala
   1               5                   10                  15
   Leu Ala Arg Ser Leu Met Leu Cys Ala Ser Pro Ala Gly Leu Pro Arg
               20                  25                  30
   Arg Ile Gly Ala Leu Ala Leu Ser Gly Arg Ser Ser Ser Ala Ser Leu
           35                  40                  45
   Leu Val Ile Ser Ser Ala Ser Ser Arg Met
       50                  55
<210> 30
   <211> 21
   <212> PRT
   <213> Hepatitis E virus
<400> 30
   Leu Arg Cys Val Trp Met Leu Phe Pro Val Phe Met Gly Phe Pro Leu
   1               5                   10                  15
   Asp Ser Phe Ile Thr
               20
<210> 31
   <211> 17
   <212> PRT
   <213> Hepatitis E virus
<400> 31
   Leu Ala Cys Tyr Arg Leu Leu Leu Met Ala Arg His Ile Ser Leu Ser
   1               5                   10                  15
   Gln
<210> 32
   <211> 4
   <212> PRT
   <213> Hepatitis E virus
<400> 32
   Asn Gln Cys Ser
   1
<210> 33
   <211> 4
   <212> PRT
   <213> Hepatitis E virus
<400> 33
   Cys Arg Asn Ile
   1
<210> 34
   <211> 38
   <212> PRT
   <213> Hepatitis E virus
<400> 34
   Ile Asn Arg His Cys Ala Leu Pro His Gly Arg Pro Glu Pro Ala Gln
   1 5 10 15
   Gly Arg Ala Val His Thr Arg Gly Pro Leu Arg Arg Ser His Lys Ala
   20 25 30
   Leu Gln Cys Phe Pro Leu
   35
<210> 35
   <211> 20
   <212> PRT
   <213> Hepatitis E virus
<400> 35
   Cys Ser Arg Leu Ser Arg Pro Phe Tyr Pro Gly His Trp Pro Arg Thr
   1 5 10 15
   Gly Tyr Asn Leu
   20
<210> 36
   <211> 19
   <212> PRT
   <213> Hepatitis E virus
<400> 36
   Ile Val Arg Ala Ser Gly Gly His Gly Arg Glu Gly Pro Gly Trp Leu
   1 5 10 15
   Arg Arg Pro
<210> 37
   <211> 4
   <212> PRT
   <213> Hepatitis E virus
<400> 37
   Ser Leu Gln Pro
   1
<210> 38
   <211> 11
   <212> PRT
   <213> Hepatitis E virus
<400> 38
   Arg Val Gln Asp His Leu Leu Pro Glu Arg Leu
   1 5 10
<210> 39
   <211> 5
   <212> PRT.
   <213> Hepatitis E virus
<400> 39
   Gln Val His His Arg
   1 5
<210> 40
   <211> 5
   <212> PRT
   <213> Hepatitis E virus
<400> 40
   Asp His Cys Pro Trp
   1 5
<210> 41
   <211> 23
   <212> PRT
   <213> Hepatitis E virus
<400> 41
   Ser Gly Pro Gly His Leu Gly Leu Glu Gln Asp Leu Leu Arg Pro Leu
   1 5 10 15
   Trp Pro Leu Val Pro Arg Tyr
   20
<210> 42
   <211> 14
   <212> PRT
   <213> Hepatitis E virus
<400> 42
   Glu Gly Tyr Ser Gly Pro Ala Pro Ser Gly Cys Val Leu Arg
   1 5 10
<210> 43
   <211> 16
   <212> PRT
   <213> Hepatitis E virus
<400> 43
   His Arg Leu Leu Gly Gly Cys Gly Arg Ser Lys Gly Ile His Gly Val
   1 5 10 15
<210> 44
   <211> 4
   <212> PRT.
   <213> Hepatitis E virus
<400> 44
   Leu His Pro Glu
   1
<210> 45
   <211> 48
   <212> PRT
   <213> Hepatitis E virus
<400> 45
   Leu Phe Ser Gly Ser Arg Val Cys Tyr Tyr Gly Gly Val Trp Asp Ala
   1 5 10 15
   Ala Val Ala His Pro Pro Val Ser Pro Tyr Lys Val Cys Val Asp Leu
   20 25 30
   Ala Gly Pro Glu Gly Val Ser Ala Arg Val Leu Glu Glu Thr Leu Arg
   35 40 45
<210> 46
   <211> 19
   <212> PRT
   <213> Hepatitis E virus
<400> 46
   Ala Arg His Ser Ser Met Glu Tyr Cys Leu Glu Tyr Gly Arg Tyr Tyr
   1 5 10 15
   Pro Leu Leu
<210> 47
   <211> 9
   <212> PRT
   <213> Hepatitis E virus
<400> 47
   Leu Pro Arg Phe Ser Gly Gly Cys Leu
   1 5
<210> 48
   <211> 6
   <212> PRT
   <213> Hepatitis E virus
<400> 48
   Phe Asp Ser Ala Leu Gln
   1 5
<210> 49
   <211> 41
   <212> PRT
   <213> Hepatitis E virus
<400> 49
   Val Ser Ser Glu Ser Arg Ser Cys Cys Pro Asp Arg Arg Leu Trp Leu
   1 5 10 15
   Glu Val Glu Gly Arg Phe Pro Pro Asp Arg Phe Val Cys Arg Cys Cys
   20 25 30
   Gly Gly Pro Arg Pro Trp Arg Ala Pro
   35 40
<210> 50
   <211> 17
   <212> PRT
   <213> Hepatitis E virus
<400> 50
   Cys Cys Ala Leu Arg Arg Pro Ala Tyr Arg Glu Glu Leu Gly Pro Trp
   1 5 10 15
   Pro
<210> 51
   <211> 9
   <212> PRT
   <213> Hepatitis E virus
<400> 51
   Ala Gly Gly Ala Ala Pro Pro Arg Cys
   1 5
<210> 52
   <211> 28
   <212> PRT
   <213> Hepatitis E virus
<400> 52
   Phe Pro Pro Gln Ala His Glu Cys Ser Ser Asp Val Cys Gly Cys Cys
   1 5 10 15
   Phe Pro Cys Leu Trp Gly Phe Pro Trp Thr Arg Ser
   20 25
<210> 53
   <211> 9
   <212> PRT
   <213> Hepatitis E virus
<400> 53
   Pro Asp Trp His Ala Thr Gly Cys Cys
   1 5
<210> 54
   <211> 6
   <212> PRT
   <213> Hepatitis E virus
<400> 54
   Trp Gln Gly Thr Phe His
   1 5
<210> 55
   <211> 7
   <212> PRT
   <213> Hepatitis E virus
<400> 55
   Val Ser Lys Thr Ser Ala Arg
   1 5